# EUROPÄISCHE PATENTSCHRIFT

(11) **EP 3 356 334 B1**
(45) Veröffentlichungstag und Bekanntmachung des Hinweises auf die Patenterteilung: **18.12.2019**
(21) Anmeldenummer: 16767299.7
(22) Anmeldetag: 21.09.2016
(51) Int. Cl.: C07D 249/14, C07D 257/06, C07D 271/08, C07D 271/113, A01N 43/653, A01N 43/713, A01N 43/832, A01N 43/824

(54) **ACYLIERTE N-(1,2,5-OXADIAZOL-3-YL)-, N-(1,3,4-OXADIAZOL-2-YL)-, N-(TETRAZOL-5-YL)- UND N-(TRIAZOL-5-YL)-ARYLCARBONSÄUREAMIDE UND IHRE VERWENDUNG ALS HERBIZIDE**
ACYLATED N-(1,2,5-OXADIAZOL-3-YL)-, N-(1,3,4-OXADIAZOL-2-YL)-, N-(TETRAZOL-5-YL)- AND N-(TRIAZOL-5-YL)-ARYLCARBONIC ACID AMIDES AND THEIR USE AS HERBICIDES
DERIVES ACYLE DE N-(1,2,5-OXADIAZOL-3-YL)-, N-(1,3,4-OXADIAZOL-2-YL)-, N-(TETRAZOL-5-YL)- ET N-(TRIAZOL-5-YL)-ARYLAMIDE ET LEUR UTILISATION COMME DES HERBICIDES

(30) Priorität: 28.09.2015 EP 15187024
(43) Veröffentlichungstag der Anmeldung: 08.08.2018
(73) Patentinhaber: Bayer CropScience Aktiengesellschaft, 40789 Monheim am Rhein (DE)
(72) Erfinder: KÖHN, Arnim, 55270 Klein-Winternheim (DE); BRAUN, Ralf, 76857 Ramberg (DE); WALDRAFF, Christian, 61118 Bad Vilbel (DE); AHRENS, Hartmut, 63225 Langen (DE); VAN ALMSICK, Andreas, 61184 Karben (DE); LEHR, Stefan, 65835 Liederbach (DE); LINDELL, Stephen David, 65817 Eppstein (DE); DIETRICH, Hansjörg, 65835 Liederbach am Taunus (DE); ROSINGER, Christopher, Hugh, 65719 Hofheim (DE); GATZWEILER, Elmar, 61231 Bad Nauheim (DE)
(74) Vertreter: BIP Patents
(86) Internationale Anmeldenummer: PCT/EP2016/072345
(87) Internationale Veröffentlichungsnummer: WO 2017/055146

(56) Entgegenhaltungen:
- WO-A1-2013/087577
- ALY, A. S. ET AL: "Synthesis and reactions of some derivatives of 2-amino-5-(4-pyridyl)-1,3,4-oxadiazole", EGYPTIAN JOURNAL OF PHARMACEUTICAL SCIENCES , 33(3-4), 699-711 CODEN: EJPSBZ; ISSN: 0301-5068, 1992, XP009187224,
- SHCHIPANOV, V. P.: "Tautomerism of 5-aminotetrazole. VII. Synthesis, structure, and spectra of diacyl drivatives of 1- and 2-methyl-5-aminotetrazoles", KHIMIYA GETEROTSIKLICHESKIKH SOEDINENII , (5), 923-6 CODEN: KGSSAQ; ISSN: 0132-6244, 1969, XP009187214,
- GEHLEN, HEINZ ET AL: "2-Amino-1,3,4-oxadiazoles. VIII. Formation of 2-amino-5-aminoalkyl-1,3,4-oxadiazoles and their conversion into 1,2,4-triazoles and triazolones", JUSTUS LIEBIGS ANNALEN DER CHEMIE , 651, 128-32 CODEN: JLACBF; ISSN: 0075-4617, 1962, XP009187222,

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

WO 2011/035874 A1 beschreibt N-(1,2,5-Oxadiazol-3-yl)-arylcarbonsäureamide als Herbizide. Aus WO 2012/028579 A1 sind herbizid wirksame N-(Tetrazol-5-yl)- und N-(Triazol-5-yl)-arylcarbonsäureamide bekannt. WO 2012/126932 A1 beschreibt N-(1,3,4-Oxadiazol-2-yl)-arylcarbonsäureamide als Herbizide.

Aus AL Y, A. S. ET AL: "Synthesis and reactions of some derivatives of 2-amino-5-(4-pyridyl)-1,3,4-oxadiazole", EGYPTIAN JOURNAL OF PHARMACEUTICAL SCIENCES, 33(3-4), 699-711, 1992, ist die Verbindung N-Benzoyl-N-[5-(pyridine-4-yl)-1,3,4-oxadiazol-2-yl]benzamid bekannt.

SHCHIPANOV, V. P.: "Tautomerism of 5-aminotetrazole. VII. Synthesis, structure, and spectra of diacyl drivatives of 1- and 2-methyl-5-aminotetrazoles", KHIMIYA GETEROTSIKLICHESKIKH SOEDINENII , (5), 923-926, 1969, sind die Verbindungen N-Acetyl-N-(1-methyl-tetrazol-5-yl)-benzamid und N-Benzoyl-N-(1-methyl-tetrazol-5-yl)-benzamid bekannt.

Aus GEHLEN, HEINZ ET AL: "2-Amino-1 ,3,4-oxadiazoles. VIII. Formation of 2-amino-5-aminoalkyl-1,3,4-oxadiazoles and their conversion into 1,2,4-triazoles and triazolones", JUSTUS LIEBIGS ANNALEN DER CHEMIE, 651, 128-132, 1962, ist die Verbindung N-Benzoyl-N-{5-[4-({[-(methylsulfonyl)phenyl]amino}methyl) phenyl]-1,3,4-oxadiazol-2-yl}benzamide bekannt.

Eine herbizide Wirkung der in den drei vorstehend genannten Schriften offenbarten Verbindungen ist nicht beschrieben.

Aufgabe der vorliegenden Erfindung war die Bereitstellung weiterer herbizid wirksamer Verbindungen.

Es wurde nun gefunden, dass Benzoylamide, die am Stickstoffatom durch bestimmte Reste subsituiert sind, als Herbizide besonders gut geeignet sind.

Ein Gegenstand der vorliegenden Erfindung sind somit N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide oder deren Salze der Formel (I) 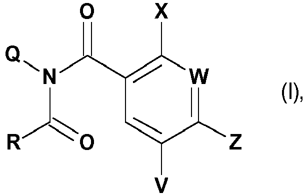 worin die Symbole und Indizes folgende Bedeutungen haben:
R bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Cycloalkyl-(C₁-C₆)-alkyl, (C₃-C₆)-Halogencycloalkyl-(C₁-C₆)-alkyl, (C₁-C₆)-AlkylS(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², COOR¹, CON(R¹)₂, oder jeweils durch s Reste aus der Gruppe bestehend aus X, Y, Z und V substituiertes Phenyl, Heteroaryl, Heterocyclyl oder Benzyl,
W bedeutet N oder CY,
X und Z bedeuten unabhängig voneinander jeweils Wasserstoff, Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, CH=NOR¹, (C₁-C₆)-Alkyl-CH=NOR¹, (C₁-C₆)-Alkyl-O-N=C(R¹)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die 6 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
   oder
Y und Z bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils s Stickstoffatome, n Sauerstoffatome, n Schwefelatome und n Elemente S(O), S(O)₂, C=N-R¹⁷, C(OR¹⁷)₂, C[-O-(CH₂)₂-O-] oder C(O) als Ringglieder umfasst,
dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₁-C₆)-Alkoxy, Phenoxy, Halogen-(C₁-C₆)-alkoxy, (C₃-C₈)-Cycloalkyl, (C₂-C₈)-Alkoxyalkyl und Phenyl substituiert sind,
dessen Stickstoffatome durch n Reste aus der Gruppe bestehend aus (C₁-C₆)-Alkyl und Phenyl substituiert sind,
und worin die vorstehend genannten Phenylreste durch s Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
V bedeutet Wasserstoff, Nitro, Halogen, Cyano, (C₁-C₄)-Alkyl, (C₁-C₄)-Halogenalkyl, OR¹, S(O)ₙR²,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₃)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
R⁵ bedeutet (C₁-C₄)-Alkyl,
n bedeutet 0, 1 oder 2,
s bedeutet 0, 1, 2 oder 3,
Q bedeutet einen Rest Q1, Q2, Q3 oder Q4 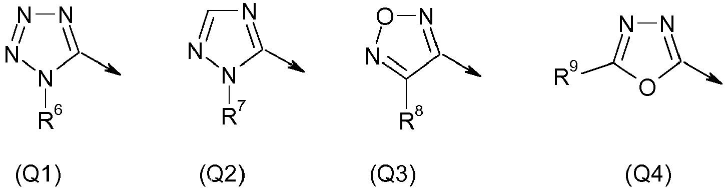
R⁶ und R⁷ bedeuten unabhängig voneinander (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl oder D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt,
   oder
R⁶ und R⁷ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁸ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfenyl, Methylsulfinyl, Methylsulfonyl, (C₁-C₆)-Alkylcarbonylamino, (C₁-C₆)-Alkoxycarbonylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁹ bedeutet Wasserstoff, (C₁-C₆)-Alkyl, R¹³O-(C₁-C₆)-Alkyl, CH₂R¹⁴, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹³, NHR¹³, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹¹ bedeutet (C₁-C₆)-Alkyl, (C₂-C₆)-Alkenyl, (C₂-C₆)-Alkinyl oder Phenyl,
R¹² bedeutet (C₁-C)-Alkyl,
R¹³ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₃)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁵-Heteroaryl oder (C₁-C₆)-Alkyl-NR¹⁵-Heterocyclyl, wobei die 21 letztgenannten Reste durch s Reste aus der Gruppe bestehend aus Cyano, Halogen, Nitro, Rhodano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ und (C₁-C₄)-Alkoxy-(C₂-C₆)-alkoxycarbonyl substituiert sind, und wobei Heterocyclyl n Oxogruppen trägt,
R¹⁴ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl;
R¹⁵ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁶ bedeutet (C₁-C₆)-Alkyl, (C₂-C₆)-Alkenyl oder (C₂-C₆)-Alkinyl,
R¹⁷ bedeutet (C₁-C₆)-Alkyl, Halogen-(C₁-C₆)-alkyl, (C₁-C₆)-Alkoxy und Halogen-(C₁-C₆)-alkoxy,
s bedeutet 0, 1, 2 oder 3,
n bedeutet 0, 1 oder 2,
D bedeutet O, S, oder NR¹¹,
mit der Maßgabe, daß V, X, Y und Z nicht gleichzeitig Wasserstoff bedeuten.
Bevorzugt sind Verbindungen der allgemeinen Formel (I), worin
R bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Cycloalkyl-(C₁-C₆)-alkyl, (C₃-C₆)-Halogencycloalkyl-(C₁-C₆)-alkyl, (C₁-C₆)-AlkylS(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², COOR¹, CON(R¹)₂, oder jeweils durch s Reste aus der Gruppe bestehend aus X, Y, Z und V substituiertes Phenyl, Heteroaryl, Heterocyclyl oder Benzyl,
W bedeutet N oder CY,
X und Z bedeuten unabhängig voneinander jeweils Wasserstoff, Halogen, (C₁-C₆)-Alkyl, (C₁-C₆)-Halogenalkyl, (C₂-C₆)-Alkenyl, (C₃-C₆)-Cycloalkyl, (C₃-C₆)-Halogencycloalkyl, OR¹, S(O)ₙR², SO₂N(R¹)₂, NR¹SO₂R², NR¹COR¹, (C₁-C₆)-AlkylS(O)ₙR², (C₁-C₆)-Alkyl-OR¹, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, (C₂-C₆)-Alkenyl, COR¹, CO₂R¹,OCO₂R¹, NR¹CO₂R¹, C(O)N(R¹)₂, NR¹C(O)N(R¹)₂, OC(O)N(R¹)₂, C(O)N(R¹)OR¹, NR¹SO₂R², NR¹COR¹, OR¹, S(O)ₙR², SO₂N(R¹)₂,(C₁-C₆)-Alkyl-S(O)ₙR², (C₁-C₆)-Alkyl-OR¹, (C₁-C₆)-Alkyl-OCOR¹, (C₁-C₆)-Alkyl-CO₂R¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, CH=NOR¹, (C₁-C₆)-Alkyl-CH=NOR¹, (C₁-C₆)-Alkyl-Heteroaryl, (C₁-C₆)-Alkyl-Heterocyclyl, Heteroaryl oder Heterocyclyl, wobei die 4 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
V bedeutet Wasserstoff, Cl, OMe, Methyl oder Ethyl,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₃)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
n bedeutet 0, 1 oder 2,
s bedeutet 0, 1, 2 oder 3,
Q bedeutet einen Rest Q1, Q2, Q3 oder Q4 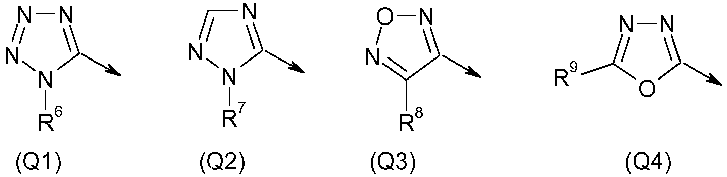
R⁶ und R⁷ bedeuten unabhängig voneinander (C₁-C₆)-Alkyl, Halogen-(C₁-C₆)-alkyl, (C₂-C₆)-Alkenyl, wobei diese 3 vorstehend genannten Reste jeweils durch s (C₁-C₆)-Alkoxy-Reste substituiert sind,
R⁸ bedeutet Chlor, Methyl, Methoxymethyl, Amino oderAcetylamino,
R⁹ bedeutet Methyl, Ethyl, Methoxymethyl oder Methoxyethyl,
mit der Maßgabe, daß V, X, Y und Z nicht gleichzeitig Wasserstoff bedeuten.
Besonders bevorzugt sind Verbindungen der allgemeinen Formel (I), worin
R bedeutet Methyl oder jeweils durch s Reste aus der Gruppe bestehend aus X, Y und Z substituiertes Phenyl,
W bedeutet CY,
X bedeutet F, Cl, Br, Methyl, Ethyl, Cyclopropyl, Trifluormethyl, Methoxy, Methoxymethyl, Methoxyethoxymethyl, SMe oder SO₂Me,
Z bedeutet Wasserstoff, F, Cl, Br, I, Methyl, Ethyl, Trifluormethyl, Difluormethyl, Pentafluorethyl, Methylsulfonyl oder Ethylsulfonyl,
Y bedeutet Wasserstoff, SMe, S(O)Me, SO₂Me, SEt, S(O)Et, SO₂Et, CH₂OMe, CH₂OEt, CH₂OCH₂CF₃, ,CH₂SMe, CH₂S(O)Me, CH₂SO₂Me, Vinyl, C(O)Me, C(O)Et, C(O)cPr, CO₂Me, CHN=OMe, 4,5-dihydro-1,2-oxazol-3-yl, 5-methyl-4,5-dihydro-1,2-oxazol-3-yl, 5-methyl-4,5-dihydro-1,2-oxazol-3-yl, 5-cyanomethyl-4,5-dihydro-1,2-oxazol-3-yl, 4,5-dihydro-1,2-oxazol-5-yl, 3-methyl-4,5-dihydro-1,2-oxazol-5-yl, 1H-pyrazol-1-yl, 1H-1,2,3-triazol-1-yl, 2H-1,2,3-triazol-2-yl, 1H-1,2,4-triazol-1-yl, pyrolidin-2-on-1-yl, morpholin-3-on-4-yl, OMe, OEt, OnPr, OCH₂cPr, OCH₂CH₂F, OCH₂CH₂OMe oder OCH₂CH₂CH₂OMe,
V bedeutet Wasserstoff,
Q bedeutet einen Rest Q1, Q2, Q3 oder Q4 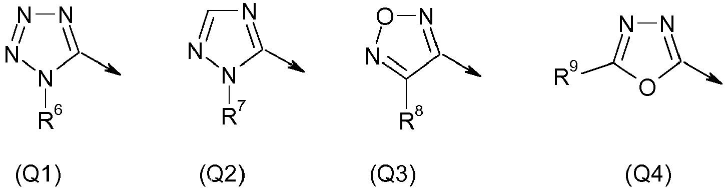
   - R⁶: bedeutet Methyl oder Ethyl,
   - R⁷: bedeutet Methyl,
   - R⁸: bedeutet Chlor oder Methyl,
   - R⁹: bedeutet Methyl,
   - s: bedeutet 0,1,2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass R¹ eine COOH-Gruppe oder eine Sulfonamid-Gruppe -NHSO₂- enthält. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R'" jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Erfindungsgemäße Verbindungen können beispielsweise nach der in Schema 1 angegebenen Methode durch Umsetzung Arylcarbonsäureamids (II) mit einem Säurechlorid oder ein Anhydrid der allgemeinen Formel (III) hergestellt werden:

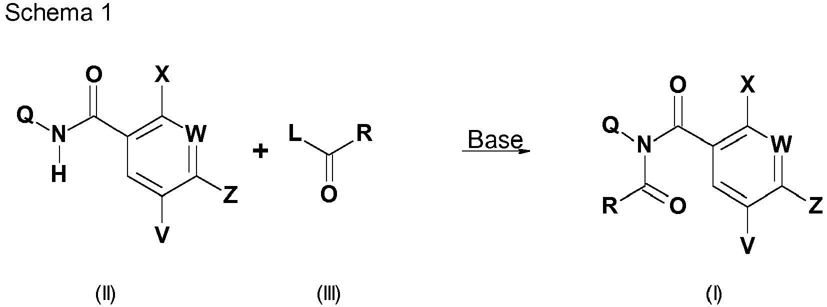

Die Arylcarbonsäureamide der Formel (II) sind grundsätzlich bekannt und können beispielsweise gemäß den in WO2011/035874 A1, WO2012/123416 A1, WO 2012/028579 A1 und WO2012/126932 beschriebenen Methoden hergestellt werden.

Erfindungsgemäße Verbindungen mit zwei identischen Acylresten können beispielsweise auch nach der in Schema 2 angegebenen Methode durch Umsetzung eines Amins der Formel (IV) mit einem Säurechlorid (V), hergestellt werden: 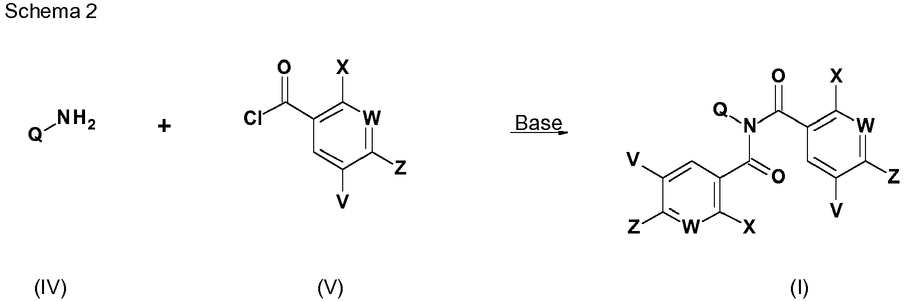 Erfindungsgemäße Verbindungen mit zwei identischen Acylresten können auch nach der in Schema 3 angegebenen Methode durch Umsetzung eines Amins der Formel (IV) mit einer Säure der Formel (VI) hergestellt werden:

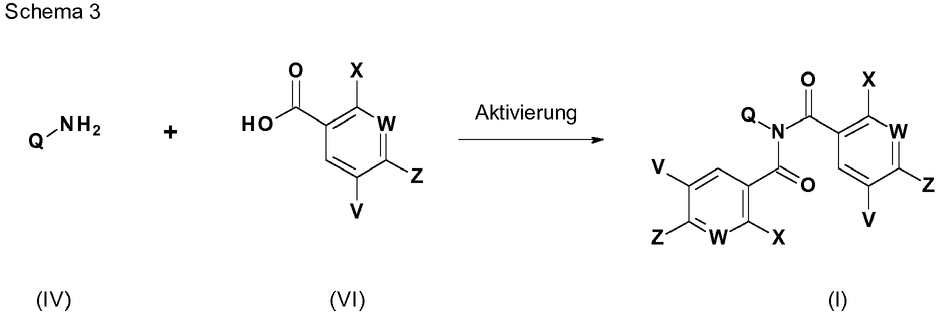

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Die Benzoesäurechloride der Formel (V) beziehungsweise die ihnen zugrunde liegenden Benzoesäuren der Formel (VI) sind grundsätzlich bekannt und können beispielsweise gemäß den in US 6,376,429 B1, EP 1 585 742 A1 und EP 1 202 978 A1 beschriebenen Methoden hergestellt werden.

Kollektionen aus Verbindungen der Formel (I), die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die erfindungsgemäßen Verbindungen der Formel (I), im folgenden als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 2-(Methylsulfonyl)-4-trifluormethyl-N-acetyl-N-(1-methyl-tetrazol-5-yl)-benzamid (Tabellenbeispiel Nr. 13-1)

Zu 175 mg (0.5 mmol) 2-(Methylsulfonyl)-4-trifluormethyl-N-(1-methyl-tetrazol-5-yl)-benzamid in 5 ml THF werden unter Argon bei 0°C 14 mg (0.55 mmol) Natriumhydrid (95%ig) gegeben. Nach 10 min Rühren werden 47 mg (0.6 mmol) Acetylchlorid zugegeben. Das Gemisch wird 1h bei 0°C gerührt und anschließend mit 2 ml gesättigter Natriumbicarbonatlösung gequencht. Die Mischung wird mit Essigester und Wasser verdünnt und die organische Phase mit gesättigter Natriumbicarbonatlösung und Kochsalzlösung gewaschen, über Na2S04 getrocknet und eingedampft. Ausbeute 143 mg (73%).

### 2. Synthese von 3-(Ethoxycarbonyl)-2-methyl-4-(methylsulfanyl)-N-(3-(Ethoxycarbonyl)-2-methyl-4-(methylsulfanyl)benzoyl)-N-(1-methyl-tetrazol-5-yl)-benzamid (Tabellenbeispiel Nr. 1-453)

Zu 525 mg (2.06 mmol) von 3-(Ethoxycarbonyl)-2-methyl-4-(methylsulfanyl)-benzoesäure, 313 mg (3.09 mmol) 1-Methyl-5-aminotetrazol in 3 ml Pyridin werden unter Eisbadkühlung 367 mg (3.09 mmol) Thionylchlorid gegeben. Das Gemisch wird 3 Tage bei RT gerührt, anschließend werden 0.2 ml Wasser zugegeben, 30 min bei RT gerührt und mit EE und 2N HCl versetzt. Die abgetrennte organische Phase wird nochmals mit 2N HCl und ges. Kochsalzlösung gewaschen, über Na2S04 getrocknet, eingedampft und mittels RP-HPLC (Acetonitril/Wassser) gereinigt. Ausbeute 112 mg (9%).

Erhalten wurden zusätzlich 58 mg (11% Ausbeute) von 3-(Ethoxycarbonyl)-2-methyl-4-(methylsulfanyl)-N-(1-methyl-tetrazol-5-yl)-benzamid.

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt

### Die verwendeten Abkürzungen bedeuten:

Et = Ethyl Me = Methyl n-Pr = n-Propyl i-Pr = Isopropyl c-Pr = Cyclopropyl Ph = Phenyl

**Tabelle 1: Erfindungsgemäße Verbindungen der Formel (I), worin Q für Q1, W für C-Y, R für substituiertes Phenyl und R⁶ für Methyl steht.**

| | | | | | |
|---|---|---|---|---|---|
| 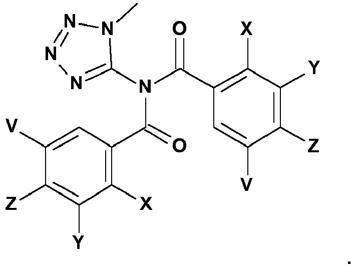 | | | | | |

| Nr. | X | Y | Z | V | Physikalische Daten (¹H-NMR, DMSO-d₆, 400 MHz) |
|---|---|---|---|---|---|
| 1-1 | F | H | Cl | H | |
| 1-2 | F | H | SO₂Me | H | |
| 1-3 | F | H | SO₂Et | H | |
| 1-4 | F | H | CF₃ | H | |
| 1-5 | F | H | NO₂ | H | |
| 1-6 | Cl | H | Br | H | |
| 1-7 | Cl | H | SMe | H | |
| 1-8 | Cl | H | SOMe | H | |
| 1-9 | Cl | H | SO₂Me | H | |
| 1-10 | Cl | H | SO₂CH₂Cl | H | |
| 1-11 | Cl | H | SEt | H | |
| 1-12 | Cl | H | SO₂Et | H | |
| 1-13 | Cl | H | CF₃ | H | |
| 1-14 | Cl | H | NO₂ | H | |
| 1-15 | Cl | H | pyrazol-1-yl | H | |
| 1-16 | Cl | H | 1H-1,2,4-triazol-1-yl | H | |
| 1-17 | Br | H | Cl | H | |
| 1-18 | Br | H | Br | H | |
| 1-19 | Br | H | SO₂Me | H | |
| 1-20 | Br | H | SO₂Et | H | |
| 1-21 | Br | H | CF₃ | H | |
| 1-22 | SO₂Me | H | Cl | H | |
| 1-23 | SO₂Me | H | Br | H | |
| 1-24 | SO₂Me | H | SMe | H | |
| 1-25 | SO₂Me | H | SOMe | H | |
| 1-26 | SO₂Me | H | SO₂Me | H | |
| 1-27 | SO₂Me | H | SO₂Et | H | |
| 1-28 | SO₂Me | H | CF₃ | H | 8.38 (s,2H), 8.30 (d,2H), 8.21 (brs,2H), 4.20 (s,3H), 3.41 (s,6H), 2.22 (s,3H) |
| 1-29 | SO₂Et | H | Cl | H | |
| 1-30 | SO₂Et | H | Br | H | |
| 1-31 | SO₂Et | H | SMe | H | |
| 1-32 | SO₂Et | H | SOMe | H | |
| 1-33 | SO₂Et | H | SO₂Me | H | |
| 1-34 | SO₂Et | H | CF₃ | H | |
| 1-35 | NO₂ | H | F | H | |
| 1-36 | NO₂ | H | Cl | H | |
| 1-37 | NO₂ | H | Br | H | |
| 1-38 | NO₂ | H | I | H | |
| 1-39 | NO₂ | H | CN | H | |
| 1-40 | NO₂ | H | SO₂Me | H | |
| 1-41 | NO₂ | H | SO₂Et | H | |
| 1-42 | NO₂ | H | CF₃ | H | |
| 1-43 | Me | H | Cl | H | |
| 1-44 | Me | H | Br | H | |
| 1-45 | Me | H | SMe | H | |
| 1-46 | Me | H | SO₂Me | H | |
| 1-47 | Me | H | SO₂CH₂Cl | H | |
| 1-48 | Me | H | SEt | H | |
| 1-49 | Me | H | SO₂Et | H | |
| 1-50 | Me | H | CF₃ | H | |
| 1-51 | CH₂SO₂Me | H | CF₃ | H | |
| 1-52 | Et | H | Cl | H | |
| 1-53 | Et | H | Br | H | |
| 1-54 | Et | H | SMe | H | |
| 1-55 | Et | H | SO₂Me | H | |
| 1-56 | Et | H | SO₂CH₂Cl | H | |
| 1-57 | Et | H | SEt | H | |
| 1-58 | Et | H | SO₂Et | H | |
| 1-59 | Et | H | CF₃ | H | |
| 1-60 | CF₃ | H | Cl | H | |
| 1-61 | CF₃ | H | Br | H | |
| 1-62 | CF₃ | H | SO₂Me | H | |
| 1-63 | CF₃ | H | SO₂Et | H | |
| 1-64 | CF₃ | H | CF₃ | H | |
| 1-65 | NO₂ | NH₂ | F | H | |
| 1-66 | NO₂ | NHMe | F | H | |
| 1-67 | NO₂ | NMe₂ | F | H | |
| 1-68 | NO₂ | Me | Cl | H | |
| 1-69 | NO₂ | NH₂ | Cl | H | |
| 1-70 | NO₂ | NHMe | Cl | H | |
| 1-71 | NO₂ | NMe₂ | Cl | H | |
| 1-72 | NO₂ | NH₂ | Br | H | |
| 1-73 | NO₂ | NHMe | Br | H | |
| 1-74 | NO₂ | NMe₂ | Br | H | |
| 1-75 | NO₂ | NH₂ | CF₃ | H | |
| 1-76 | NO₂ | NMe₂ | CF₃ | H | |
| 1-77 | NO₂ | NH₂ | SO₂Me | H | |
| 1-78 | NO₂ | NH₂ | SO₂Et | H | |
| 1-79 | NO₂ | NHMe | SO₂Me | H | |
| 1-80 | NO₂ | NMe₂ | SO₂Me | H | |
| 1-81 | NO₂ | NMe₂ | SO₂Et | H | |
| 1-82 | NO₂ | NH₂ | 1H-1,2,4-triazol-1-yl | H | |
| 1-83 | NO₂ | NHMe | 1H-1,2,4-triazol-1-yl | H | |
| 1-84 | NO₂ | NMe₂ | 1H-1,2,4-triazol-1-yl | H | |
| 1-85 | Me | SMe | H | H | ¹H-NMR (400,1 MHz, CDCl₃): 7,518 (0,8); 7,325 (1,0); 7,320 (1,2); 7,310 (0,4); 7,306 (2,3); 7,301 (2,3); 7,280 (2,0); 7,272 (0,3); 7,2694 (0,4); 7,2687 (0,4); 7,268 (0,5); 7,266 (0,7); 7,265 (0,9); 7,259 (133,8); 7,242 (1,8); 7,170 (1,5); 7,166 (1,6); 7,150 (1,1); 7,146 (1,1); 6,995 (0,7); 5,298 (1,0); 4,107 (9,8); 2,446 (16,0); 1,527 (33,0); 0,008 (1,6) |
| 1-86 | Me | SOMe | H | H | 8.10-8.06 (m, 2H), 7.69-7.60 (m, 4H), 4.10 (s, 3H), 2.79 (s, 6H), |
| 1-87 | Me | SO₂Me | H | H | |
| 1-88 | Me | SEt | H | H | |
| 1-89 | Me | SOEt | H | H | |
| 1-90 | Me | SO₂Et | H | H | |
| 1-91 | Me | S(CH₂)₂OMe | H | H | |
| 1-92 | Me | SO(CH₂)₂OMe | H | H | |
| 1-93 | Me | SO₂(CH₂)₂OMe | H | H | |
| 1-94 | Me | F | F | H | |
| 1-95 | Me | F | SMe | H | |
| 1-96 | Me | SEt | F | H | |
| 1-97 | Me | SOEt | F | H | |
| 1-98 | Me | SO₂Et | F | H | |
| 1-99 | Me | Me | Cl | H | |
| 1-100 | Me | F | Cl | H | |
| 1-101 | Me | Cl | Cl | H | |
| 1-102 | Me | NH₂ | Cl | H | |
| 1-103 | Me | NHMe | Cl | H | |
| 1-104 | Me | NMe₂ | Cl | H | |
| 1-105 | Me | O(CH₂)₂OMe | Cl | H | |
| 1-106 | Me | O(CH₂)₃OMe | Cl | H | |
| 1-107 | Me | O(CH₂)₄OMe | Cl | H | |
| 1-108 | Me | OCH₂CONMe₂ | Cl | H | |
| 1-109 | Me | O(CH₂)₂-CO-NMe₂ | Cl | H | |
| 1-110 | Me | O(CH₂)₂-NH(CO)NMe₂ | Cl | H | |
| 1-111 | Me | O(CH₂)₂-NH(CO)NHCO₂Et | Cl | H | |
| 1-112 | Me | O(CH₂)₂-NHCO₂Me | Cl | H | |
| 1-113 | Me | OCH₂-NHSO₂cPr | Cl | H | |
| 1-114 | Me | O(CH₂)-5-2,4-dimethyl-2,4-dihydro-3H-1,2,4-triazol-3-on | Cl | H | |
| 1-115 | Me | O(CH₂)-3,5-dimethyl-1,2-oxazol-4-yl | Cl | H | |
| 1-116 | Me | SMe | Cl | H | |
| 1-117 | Me | SOMe | Cl | H | |
| 1-118 | Me | SO₂Me | Cl | H | |
| 1-119 | Me | SEt | Cl | H | |
| 1-120 | Me | SOEt | Cl | H | |
| 1-121 | Me | SO₂Et | Cl | H | |
| 1-122 | Me | S(CH₂)₂OMe | Cl | H | |
| 1-123 | Me | SO(CH₂)₂OMe | Cl | H | |
| 1-124 | Me | SO₂(CH₂)₂OMe | Cl | H | |
| 1-125 | Me | NH₂ | Br | H | |
| 1-126 | Me | NHMe | Br | H | |
| 1-127 | Me | NMe₂ | Br | H | |
| 1-128 | Me | OCH₂(CO)NMe₂ | Br | H | |
| 1-129 | Me | O(CH₂)-5-pyrrolidin-2-on | Br | H | |
| 1-130 | Me | SMe | Br | H | |
| 1-131 | Me | SOMe | Br | H | |
| 1-132 | Me | SO₂Me | Br | H | |
| 1-133 | Me | SEt | Br | H | |
| 1-134 | Me | SOEt | Br | H | |
| 1-135 | Me | SO₂Et | Br | H | |
| 1-136 | Me | SMe | I | H | |
| 1-137 | Me | SOMe | I | H | |
| 1-138 | Me | SO₂Me | I | H | |
| 1-139 | Me | SEt | I | H | |
| 1-140 | Me | SOEt | I | H | |
| 1-141 | Me | SO₂Et | I | H | |
| 1-142 | Me | Cl | CF₃ | H | |
| 1-143 | Me | SMe | CF₃ | H | |
| 1-144 | Me | SOMe | CF₃ | H | |
| 1-145 | Me | SO₂Me | CF₃ | H | |
| 1-146 | Me | SEt | CF₃ | H | |
| 1-147 | Me | SOEt | CF₃ | H | |
| 1-148 | Me | SO₂Et | CF₃ | H | |
| 1-149 | Me | S(CH₂)₂OMe | CF₃ | H | |
| 1-150 | Me | SO(CH₂)₂OMe | CF₃ | H | |
| 1-151 | Me | SO₂(CH₂)₂OMe | CF₃ | H | |
| 1-152 | Me | Me | SO₂Me | H | |
| 1-153 | Me | 4,5-dihydro-1,2-oxazol-3 yl | SO₂Me | H | |
| 1-154 | Me | 4,5-dihydro-1,2-oxazol-3 yl | SO₂Et | H | |
| 1-155 | Me | 5-cyanomethyl-4,5-dihydro-1,2-oxazol-3-yl | SO₂Me | H | |
| 1-156 | Me | 5-cyanomethyl-4,5-dihydro-1,2-oxazol-3-yl | SO₂Et | H | |
| 1-157 | Me | NH₂ | SO₂Me | H | |
| 1-158 | Me | NHMe | SO₂Me | H | |
| 1-159 | Me | NMe₂ | SO₂Me | H | |
| 1-160 | Me | NH(CH₂)₂OMe | SO₂Me | H | |
| 1-161 | Me | pyrazol-1-yl | SO₂Me | H | |
| 1-162 | Me | OH | SO₂Me | H | |
| 1-163 | Me | OMe | SO₂Me | H | |
| 1-164 | Me | OMe | SO₂Et | H | |
| 1-165 | Me | OEt | SO₂Me | H | |
| 1-166 | Me | OEt | SO₂Et | H | |
| 1-167 | Me | O-i-Pr | SO₂Me | H | |
| 1-168 | Me | O-i-Pr | SO₂Et | H | |
| 1-169 | Me | O(CH₂)₂OMe | SO₂Me | H | |
| 1-170 | Me | O(CH₂)₂OMe | SO₂Et | H | |
| 1-171 | Me | O(CH₂)₃OMe | SO₂Me | H | |
| 1-172 | Me | O(CH₂)₃OMe | SO₂Et | H | |
| 1-173 | Me | O(CH₂)₄OMe | SO₂Me | H | |
| 1-174 | Me | O(CH₂)₄OMe | SO₂Et | H | |
| 1-175 | Me | O(CH₂)₂NHSO2Me | SO₂Me | H | |
| 1-176 | Me | O(CH₂)₂NHSO2Me | SO₂Et | H | |
| 1-177 | Me | OCH₂(CO)NMe₂ | SO₂Me | H | |
| 1-178 | Me | OCH₂(CO)NMe₂ | SO₂Et | H | |
| 1-179 | Me | [1,4]dioxan-2-yl-methoxy | SO₂Me | H | |
| 1-180 | Me | [1,4]dioxan-2-yl-methoxy | SO₂Et | H | |
| 1-181 | Me | O(CH₂)₂-O(3,5-di-methoxypyrimidin-2-yl | SO₂Me | H | |
| 1-182 | Me | Cl | SO₂Me | H | |
| 1-183 | Me | SMe | SO₂Me | H | |
| 1-184 | Me | SOMe | SO₂Me | H | |
| 1-185 | Me | SO₂Me | SO₂Me | H | |
| 1-186 | Me | SO₂Me | SO₂Et | H | |
| 1-187 | Me | SEt | SO₂Me | H | |
| 1-188 | Me | SOEt | SO₂Me | H | |
| 1-189 | Me | SO₂Et | SO₂Me | H | |
| 1-190 | Me | S(CH₂)₂OMe | SO₂Me | H | |
| 1-191 | Me | SO(CH₂)₂OMe | SO₂Me | H | |
| 1-192 | Me | SO₂(CH₂)₂OMe | SO₂Me | H | |
| 1-193 | CH₂SMe | OMe | SO₂Me | H | |
| 1-194 | CH₂OMe | OMe | SO₂Me | H | |
| 1-195 | CH₂O(CH₂)₂OMe | NH(CH₂)₂OEt | SO₂Me | H | |
| 1-196 | CH₂O(CH₂)₂OMe | NH(CH₂)₃OEt | SO₂Me | H | |
| 1-197 | CH₂O(CH₂)₃OMe | OMe | SO₂Me | H | |
| 1-198 | CH₂O(CH₂)₂OMe | NH(CH₂)₂OMe | SO₂Me | H | |
| 1-199 | CH₂O(CH₂)₂OMe | NH(CH₂)₃OMe | SO₂Me | H | |
| 1-200 | Et | SMe | Cl | H | |
| 1-201 | Et | SO₂Me | Cl | H | |
| 1-202 | Et | SMe | CF₃ | H | |
| 1-203 | Et | SO₂Me | CF₃ | H | |
| 1-204 | Et | F | SO₂Me | H | |
| 1-205 | Et | NH(CH₂)₂OMe | SO₂Me | H | |
| 1-206 | i-Pr | SO₂Me | CF₃ | H | ¹H-NMR (400,1 MHz, CDCl₃): 7,821 (1,6); 7,801 (1,9); 7,593 (0,8); 7,575 (0,7); 7,263 (18,6); 7,260 (18,8); 5,301 (6,9); 5,298 (7,1); 4,312 (0,3); 4,295 (0,8); 4,277 (1,1); 4,260 (0,8); 4,242 (0,4); 4,133 (0,4); 4,131 (0,5); 4,120 (8,4); 4,117 (8,5); 3,335 (15,8); 3,334 (16,0); 3,266 (1,0); 3,264 (1,0); 3,196 (0,8); 3,194 (0,8); 2,046 (1,5); 2,044 (1,5); 1,714 (0,4); 1,643 (0,4); 1,640 (0,5); 1,625 (0,4); 1,623 (0,4); 1,521 (12,5); 1,503 (12,3); 1,395 (0,5); 1,393 (0,5); 1,378 (0,4); 1,375 (0,4); 1,294 (0,5); 1,292 (0,5); 1,279 (0,6); 1,277 (0,9); 1,262 (1,0); 1,259 (1,1); 1,244 (0,4); 1,241 (0,5); 0,003 (8,2); 0,000 (8,9) |
| 1-207 | cPr | SO₂Me | CF₃ | H | |
| 1-208 | CF₃ | O(CH₂)₂OMe | F | H | |
| 1-209 | CF₃ | O(CH₂)₃OMe | F | H | |
| 1-210 | CF₃ | OCH₂CONMe₂ | F | H | |
| 1-211 | CF₃ | [1,4]dioxan-2-yl-methoxy | F | H | |
| 1-212 | CF₃ | O(CH₂)₂OMe | Cl | H | |
| 1-213 | CF₃ | O(CH₂)₃OMe | Cl | H | |
| 1-214 | CF₃ | OCH₂CONMe₂ | Cl | H | |
| 1-215 | CF₃ | [1,4]dioxan-2-yl-methoxy | Cl | H | |
| 1-216 | CF₃ | O(CH₂)₂OMe | Br | H | |
| 1-217 | CF₃ | O(CH₂)₃OMe | Br | H | |
| 1-218 | CF₃ | OCH₂CONMe₂ | Br | H | |
| 1-219 | CF₃ | [1,4]dioxan-2-yl-methoxy | Br | H | |
| 1-220 | CF₃ | O(CH₂)₂OMe | I | H | |
| 1-221 | CF₃ | O(CH₂)₃OMe | I | H | |
| 1-222 | CF₃ | OCH₂CONMe₂ | I | H | |
| 1-223 | CF₃ | [1,4]dioxan-2-yl-methoxy | I | H | |
| 1-224 | CF₃ | F | SO₂Me | H | |
| 1-225 | CF₃ | F | SO₂Et | H | |
| 1-226 | CF₃ | O(CH₂)₂OMe | SO₂Me | H | |
| 1-227 | CF₃ | O(CH₂)₂OMe | SO₂Et | H | |
| 1-228 | CF₃ | O(CH₂)₃OMe | SO₂Me | H | |
| 1-229 | CF₃ | O(CH₂)₃OMe | SO₂Et | H | |
| 1-230 | CF₃ | OCH₂CONMe₂ | SO₂Me | H | |
| 1-231 | CF₃ | OCH₂CONMe₂ | SO₂Et | H | |
| 1-232 | CF₃ | [1,4]dioxan-2-yl-methoxy | SO₂Me | H | |
| 1-233 | CF₃ | [1,4]dioxan-2-yl-methoxy | SO₂Et | H | |
| 1-234 | F | SMe | CF₃ | H | |
| 1-235 | F | SOMe | CF₃ | H | |
| 1-236 | Cl | Me | Cl | H | |
| 1-237 | Cl | OCH₂CHCH₂ | Cl | H | |
| 1-238 | Cl | OCH₂CHF₂ | Cl | H | |
| 1-239 | Cl | O(CH₂)₂OMe | **Cl** | H | |
| 1-240 | Cl | OCH₂CONMe₂ | Cl | H | |
| 1-241 | Cl | O(CH₂)-5-pyrrolidin-2-on | Cl | H | |
| 1-242 | Cl | SMe | Cl | H | |
| 1-243 | Cl | SOMe | Cl | H | |
| 1-244 | Cl | SO₂Me | Cl | H | |
| 1-245 | Cl | F | SMe | H | |
| 1-246 | Cl | Cl | SO₂Me | H | |
| 1-247 | Cl | CO₂Me | SO₂Me | H | |
| 1-248 | Cl | CONMe₂ | SO₂Me | H | |
| 1-249 | Cl | CONMe(OMe) | SO₂Me | H | |
| 1-250 | Cl | CH₂OMe | SO₂Me | H | |
| 1-251 | Cl | CH₂OMe | SO₂Et | H | |
| 1-252 | Cl | CH₂OEt | SO₂Me | H | |
| 1-253 | Cl | CH₂OEt | SO₂Et | H | |
| 1-254 | Cl | CH₂OCH₂CHF₂ | SO₂Me | H | |
| 1-255 | Cl | CH₂OCH₂CF₃ | SO₂Me | H | |
| 1-256 | Cl | CH₂OCH₂CF₃ | SO₂Et | H | |
| 1-257 | Cl | CH₂OCH₂CF₂CHF₂ | SO₂Me | H | |
| 1-258 | Cl | CH₂OcPentyl | SO₂Me | H | |
| 1-259 | Cl | CH₂PO(OMe)₂ | SO₂Me | H | |
| 1-260 | Cl | 4,5-dihydro-1,2-oxazol-3 yl | SMe | H | |
| 1-261 | Cl | 4,5-dihydro-1,2-oxazol-3 yl | SO₂Me | H | |
| 1-262 | Cl | 4,5-dihydro-1,2-oxazol-3 yl | SO₂Et | H | |
| 1-263 | Cl | 5-cyanomethyl-4,5-dihydro-1,2-oxazol-3 yl | SO₂Me | H | |
| 1-264 | Cl | 5-cyanomethyl-4,5-dihydro-1,2-oxazol-3 yl | SO₂Et | H | |
| 1-265 | Cl | 5-(Methoxymethyl)-4,5-dihydro-1,2-oxazol-3 yl | SO₂Et | H | |
| 1-266 | Cl | 5-(Methoxymethyl)-5-Methyl- 4,5-dihydro-1,2-oxazol-3 yl | SO₂Et | H | |
| 1-267 | Cl | CH₂O-tetrahyd rofu ran-3-yl | SO₂Me | H | |
| 1-268 | Cl | CH₂O-tetra-hyd rofu ran-3-yl | SO₂Et | | |
| 1-269 | Cl | CH₂OCH₂-tetrahydrofuran-2-yl | SO₂Me | H | |
| 1-270 | Cl | CH₂OCH₂-tetrahydrofuran-2-yl | SO₂Et | H | |
| 1-271 | Cl | CH₂OCH₂-tetra-hydrofuran-3-yl | SO₂Me | H | |
| 1-272 | Cl | CH₂OCH₂-tetra-hydrofuran-3-yl | SO₂Et | H | |
| 1-273 | Cl | OMe | SO₂Me | H | |
| 1-274 | Cl | OMe | SO₂Et | H | |
| 1-275 | Cl | OEt | SO₂Me | H | |
| 1-276 | Cl | OEt | SO₂Et | H | |
| 1-277 | Cl | O-i-Pr | SO₂Me | H | |
| 1-278 | Cl | O-i-Pr | SO₂Et | H | |
| 1-279 | Cl | O(CH₂)₂OMe | SO₂Me | H | |
| 1-280 | Cl | O(CH₂)₄OMe | SO₂Me | H | |
| 1-281 | Cl | O(CH₂)₄OMe | SO₂Et | H | |
| 1-282 | Cl | O(CH₂)₃OMe | SO₂Me | H | |
| 1-283 | Cl | O(CH₂)₃OMe | SO₂Et | H | |
| 1-284 | Cl | O(CH₂)₂OMe | SO₂Me | H | |
| 1-285 | Cl | O(CH₂)₂OMe | SO₂Et | H | |
| 1-286 | Cl | [1,4]dioxan-2-yl-methoxy | SO₂Me | H | |
| 1-287 | Cl | [1,4]dioxan-2-yl-methoxy | SO₂Et | H | |
| 1-288 | Cl | OCH₂(CO)NMe₂ | SO₂Me | H | |
| 1-289 | Cl | OCH₂(CO)NMe₂ | SO₂Et | H | |
| 1-290 | Cl | SMe | SO₂Me | H | |
| 1-291 | Cl | SOMe | SO₂Me | H | |
| 1-292 | Br | OMe | Br | H | |
| 1-293 | Br | O(CH₂)₂OMe | Br | H | |
| 1-294 | Br | O(CH₂)₂OMe | SO₂Me | H | |
| 1-295 | Br | O(CH₂)₂OMe | SO₂Et | H | |
| 1-296 | Br | O(CH₂)₃OMe | SO₂Me | H | |
| 1-297 | Br | O(CH₂)₃OMe | SO₂Et | H | |
| 1-298 | Br | O(CH₂)₄OMe | SO₂Me | H | |
| 1-299 | Br | O(CH₂)₄OMe | SO₂Et | H | |
| 1-300 | Br | [1,4]dioxan-2-yl-methoxy | SO₂Me | H | |
| 1-301 | Br | [1,4]dioxan-2-yl-methoxy | SO₂Et | H | |
| 1-302 | I | O(CH₂)₂OMe | SO₂Me | H | |
| 1-303 | I | O(CH₂)₂OMe | SO₂Et | H | |
| 1-304 | I | O(CH₂)₃OMe | SO₂Me | H | |
| 1-305 | I | O(CH₂)₃OMe | SO₂Et | H | |
| 1-306 | I | O(CH₂)₄OMe | SO₂Me | H | |
| 1-307 | I | O(CH₂)₄OMe | SO₂Et | H | |
| 1-308 | I | [1,4]dioxan-2-yl-methoxy | SO₂Me | H | |
| 1-309 | I | [1,4]dioxan-2-yl-methoxy | SO₂Et | H | |
| 1-310 | OMe | SMe | CF₃ | H | |
| 1-311 | OMe | SOMe | CF₃ | H | |
| 1-312 | OMe | SO₂Me | CF₃ | H | |
| 1-313 | OMe | SOEt | CF₃ | H | |
| 1-314 | OMe | SO₂Et | CF₃ | H | |
| 1-315 | OMe | S(CH₂)₂OMe | CF₃ | H | |
| 1-316 | OMe | SO(CH₂)₂OMe | CF₃ | H | |
| 1-317 | OMe | SO₂(CH₂)₂OMe | CF₃ | H | |
| 1-318 | OMe | SMe | Cl | H | |
| 1-319 | OMe | SOMe | Cl | H | |
| 1-320 | OMe | SO₂Me | Cl | H | |
| 1-321 | OMe | SEt | Cl | H | |
| 1-322 | OMe | SOEt | Cl | H | |
| 1-323 | OMe | SO2Et | Cl | H | |
| 1-324 | OMe | S(CH₂)₂OMe | Cl | H | |
| 1-325 | OMe | SO(CH₂)₂OMe | Cl | H | |
| 1-326 | OMe | SO₂(CH₂)₂OMe | Cl | H | |
| 1-327 | OCH₂c-Pr | SMe | CF₃ | H | |
| 1-328 | OCH₂c-Pr | SOMe | CF₃ | H | |
| 1-329 | OCH₂c-Pr | SO₂Me | CF₃ | H | |
| 1-330 | OCH₂c-Pr | SEt | CF₃ | H | |
| 1-331 | OCH₂c-Pr | SOEt | CF₃ | H | |
| 1-332 | OCH₂c-Pr | SO₂Et | CF₃ | H | |
| 1-333 | OCH₂c-Pr | S(CH₂)₂OMe | CF₃ | H | |
| 1-334 | OCH₂c-Pr | SO(CH₂)₂OMe | CF₃ | H | |
| 1-335 | OCH₂c-Pr | SO₂(CH₂)₂OMe | CF₃ | H | |
| 1-336 | OCH₂c-Pr | SMe | Cl | H | |
| 1-337 | OCH₂c-Pr | SOMe | Cl | H | |
| 1-338 | OCH₂c-Pr | SO₂Me | Cl | H | |
| 1-339 | OCH₂c-Pr | SEt | Cl | H | |
| 1-340 | OCH₂c-Pr | SOEt | Cl | H | |
| 1-341 | OCH₂c-Pr | SO₂Et | Cl | H | |
| 1-342 | OCH₂c-Pr | S(CH₂)₂OMe | Cl | H | |
| 1-343 | OCH₂c-Pr | SO(CH₂)₂OMe | Cl | H | |
| 1-344 | OCH₂c-Pr | SO₂(CH₂)₂OMe | Cl | H | |
| 1-345 | OCH₂c-Pr | SMe | SO₂Me | H | |
| 1-346 | OCH₂c-Pr | SOMe | SO₂Me | H | |
| 1-347 | OCH₂c-Pr | SO₂Me | SO₂Me | H | |
| 1-348 | OCH₂c-Pr | SEt | SO₂Me | H | |
| 1-349 | OCH₂c-Pr | SOEt | SO₂Me | H | |
| 1-350 | OCH₂c-Pr | SO₂Et | SO₂Me | H | |
| 1-351 | OCH₂c-Pr | S(CH₂)₂OMe | SO₂Me | H | |
| 1-352 | OCH₂c-Pr | SO(CH₂)₂OMe | SO₂Me | H | |
| 1-353 | OCH₂c-Pr | SO₂(CH₂)₂OMe | SO₂Me | H | |
| 1-354 | SO₂Me | F | CF₃ | H | |
| 1-355 | SO₂Me | NH₂ | CF₃ | H | |
| 1-356 | SO₂Me | NHEt | Cl | H | |
| 1-357 | SMe | SEt | F | H | |
| 1-358 | SMe | SMe | F | H | |
| 1-359 | SMe | SMe | CF₃ | H | |
| 1-360 | SMe | SOMe | CF₃ | H | |
| 1-361 | SMe | SO₂Me | CF₃ | H | |
| 1-362 | SMe | SMe | Cl | H | |
| 1-363 | SMe | SMe | Br | H | |
| 1-364 | Cl | Ac | CF₃ | H | |
| 1-365 | Cl | Ac | SO₂Me | H | |
| 1-366 | Cl | C(O)cPr | CF₃ | H | |
| 1-367 | Cl | C(O)cPr | SO₂Me | H | |
| 1-368 | Cl | CH₂SMe | CF₃ | H | |
| 1-369 | Cl | CH₂S(O)Me | CF₃ | H | |
| 1-370 | Cl | CH₂SO₂Me | CF₃ | H | |
| 1-371 | Cl | CH₂SMe | SO₂Me | H | |
| 1-372 | Cl | CH₂S(O)Me | SO₂Me | H | |
| 1-373 | Cl | CH₂SO₂Me | SO₂Me | H | |
| 1-374 | Cl | CH=NOMe | CF₃ | H | |
| 1-375 | Cl | CH=NOMe | SO₂Me | H | |
| 1-376 | Cl | 4,5-dihydro-1,2-oxazol-5-yl, | CF₃ | H | |
| 1-377 | Cl | 4,5-dihydro-1,2-oxazol-5-yl, | SO₂Me | H | |
| 1-378 | Cl | 3-methyl-4,5-dihydro-1,2-oxazol-5-yl | CF₃ | H | |
| 1-379 | Cl | 3-methyl-4,5-dihydro-1,2-oxazol-5-yl | SO₂Me | H | |
| 1-380 | Cl | vinyl | CF₃ | H | |
| 1-381 | Cl | vinyl | SO₂Me | H | |
| 1-382 | Cl | CO₂Me | CF₃ | H | |
| 1-383 | Cl | CO₂Me | SO₂Me | H | |
| 1-384 | Cl | SMe | CF₃ | H | |
| 1-385 | Cl | S(O)Me | CF₃ | H | |
| 1-386 | Cl | SO₂Me | CF₃ | H | |
| 1-387 | Cl | SO₂Me | SO₂Me | H | |
| 1-388 | Cl | SMe | Me | H | |
| 1-389 | Cl | SOMe | Me | H | |
| 1-390 | Cl | SO₂Me | Me | H | |
| 1-391 | Cl | 1H-1,2,4-triazol-1-yl | CF₃ | H | |
| 1-392 | Cl | 1H-1,2,3-triazol-1-yl | CF₃ | H | |
| 1-393 | Cl | 2H-1,2,3-triazol-2-yl | CF₃ | H | |
| 1-394 | Cl | 1 H-pyrazol-1-yl | CF₃ | H | |
| 1-395 | Cl | 1H-4-Chlorpyrazol-1-yl | CF₃ | H | |
| 1-396 | Cl | 1 H-3-brompyrazol-1-yl | CF₃ | H | |
| 1-397 | Cl | 1H-4-trifluormethylpyrazol-1-yl | CF₃ | H | |
| 1-398 | Cl | pyrolidin-2-on-1-yl | CF₃ | H | |
| 1-399 | Cl | morpholin-3-on-4-yl | CF₃ | H | |
| 1-400 | Cl | 1,2-Thiazolidin-1,1-dioxid-2-yl | CF₃ | H | |
| 1-401 | Br | 1H-1,2,4-triazol-1-yl | CF₃ | H | |
| 1-402 | Br | 1H-1,2,3-triazol-1-yl | CF₃ | H | |
| 1-403 | Br | 2H-1,2,3-triazol-2-yl | CF₃ | H | |
| 1-404 | Br | 1 H-pyrazol-1-yl | CF₃ | H | |
| 1-405 | Br | 1H-4-Chlorpyrazol-1-yl | CF₃ | H | |
| 1-406 | Br | 1 H-3-brompyrazol-1-yl | CF₃ | H | |
| 1-407 | Br | 1H-4-trifluormethyl-pyrazol-1-yl | CF₃ | H | |
| 1-408 | Br | pyrolidin-2-on-1-yl | CF₃ | H | |
| 1-409 | Br | morpholin-3-on-4-yl | CF₃ | H | |
| 1-410 | Br | 1,2-Thiazolidin-1,1-dioxid-2-yl | CF₃ | H | |
| 1-411 | CH₂OMe | 1H-1,2,4-triazol-1-yl | CF₃ | H | |
| 1-412 | CH₂OMe | 1H-1,2,3-triazol-1-yl | CF₃ | H | |
| 1-413 | CH₂OMe | 2H-1,2,3-triazol-2-yl | CF₃ | H | |
| 1-414 | CF₃ | OCH₂CH₂F | CF₃ | H | |
| 1-415 | CF₃ | OMe | CF₃ | H | |
| 1-416 | CF₃ | SMe | CF₃ | H | |
| 1-417 | CF₃ | SOMe | CF₃ | H | |
| 1-418 | CF₃ | SO₂Me | CF₃ | H | |
| 1-419 | CF₃ | 1 H-pyrazol-1-yl | CF₃ | H | |
| 1-420 | Me | SMe | Et | H | |
| 1-421 | Me | SOMe | Et | H | |
| 1-422 | Me | SO₂Me | Et | H | |
| 1-423 | Me | 1 H-pyrazol-1-yl | Et | H | |
| 1-424 | Me | OCH₂CH₂F | Et | H | |
| 1-425 | Me | OMe | Et | H | |
| 1-426 | Me | Ac | CF₃ | H | |
| 1-427 | Me | Ac | SO₂Me | H | |
| 1-428 | Me | C(O)cPr | CF₃ | H | |
| 1-429 | Me | C(O)cPr | SO₂Me | H | |
| 1-430 | Me | CH₂SMe | CF₃ | H | |
| 1-431 | Me | CH₂S(O)Me | CF₃ | H | |
| 1-432 | Me | CH₂SO₂Me | CF₃ | H | |
| 1-433 | Me | CH₂SMe | SO₂Me | H | |
| 1-434 | Me | CH₂S(O)Me | SO₂Me | H | |
| 1-435 | Me | CH₂SO₂Me | SO₂Me | H | |
| 1-436 | Me | CH=NOMe | CF₃ | H | |
| 1-437 | Me | CH=NOMe | SO₂Me | H | |
| 1-438 | Me | 4,5-dihydro-1,2-oxazol-5-yl, | CF₃ | H | |
| 1-439 | Me | 4,5-dihydro-1,2-oxazol-5-yl, | SO₂Me | H | |
| 1-440 | Me | 3-methyl-4,5-dihydro-1,2-oxazol-5-yl | CF₃ | H | |
| 1-441 | Me | 3-methyl-4,5-dihydro-1,2-oxazol-5-yl | SO₂Me | H | |
| 1-442 | Me | vinyl | CF₃ | H | |
| 1-443 | Me | vinyl | SO₂Me | H | |
| 1-444 | Me | CO₂Me | CF₃ | H | |
| 1-445 | Me | CO₂Me | SO₂Me | H | |
| 1-446 | Me | CO₂Me | SMe | H | |
| 1-447 | Me | NH₂ | CF₃ | H | ¹H-NMR (400,0 MHz, CDCl₃): 7,519 (0,7); 7,284 (2,2); 7,260 (119,6); 6,996 (0,7); 6,821 (1,9); 6,801 (1,8); 4,281 (2,5); 4,064 (15,4); 2,422 (2,3); 2,137 (16,0); 2,045 (2,1); 1,552 (1,7); 1,277 (0,7); 1,259 (1,4); 1,241 (0,6); 0,882 (0,7); 0,008 (1,3); 0,000 (39,1); - 0,009 (1,1) |
| 1-448 | Me | NMe2 | CF₃ | H | ¹H-NMR (400,0 MHz, CDCl₃): 7,468 (0,9); 7,448 (1,4); 7,373 (1,2); 7,353 (0,8); 7,260 (55,6); 4,087 (7,4); 2,826 (1,4); 2,684 (16,0); 2,520 (0,7); 2,291 (8,3); 1,545 (2,5); 0,008 (0,6); 0,000 (19,2); - 0,009 (0,5) |
| 1-449 | Me | NHMe | CF₃ | H | ¹H-NMR (400,0 MHz, CDCl₃): 7,373 (1,7); 7,352 (2,0); 7,261 (33,1); 7,133 (1,7); 7,113 (1,5); 4,063 (16,0); 2,729 (10,7); 2,356 (14,5); 1,553 (1,5); 1,256 (1,2); 0,000 (12,2) |
| 1-450 | Me | SO₂Me | Me | H | ¹H-NMR (600, 1 MHz, CD₃CN): 7,673 (2,0); 7,659 (2,1); 7,298 (1,7); 7,285 (1,6); 4,093 (8,5); 3,043 (16,0); 2,628 (12,0); 2,615 (12,0); 2,135 (28,1); 2,103 (0,4); 2,101 (0,5); 1,956 (0,4); 1,951 (0,7); 1,948 (3,0); 1,944 (5,3); 1,939 (7,4); 1,935 (5,1); 1,931 (2,6); 0,000 (2,7) |
| 1-451 | Me | SMe | Me | H | |
| 1-452 | Me | SEt | OMe | H | ¹H-NMR (400, 1 MHz, CDCl₃): 7,579 (2,4); 7,558 (2,5); 7,260 (34,2); 6,694 (2,0); 6,672 (1,9); 4,027 (9,7); 3,876 (16,0); 2,720 (1,4); 2,701 (4,4); 2,683 (4,5); 2,665 (1,5); 2,549 (13,8); 1,546 (6,4); 1,256 (1,0); 1,042 (4,7); 1,023 (9,8); 1,005 (4,5) |
| 1-453 | Me | CO₂Et | SMe | H | 7.86 (d,2H), 7.24 (d,2H), 4.32 (q,4H), 4.16 (s,3H), 2.49 (s,6H), 2.25 (s,6H), 1.31 (t,6H) |
| 1-454 | Me | SMe | NMe₂ | H | ¹H-NMR (400,1 MHz, CDCl₃): 7,447 (1,3); 7,426 (1,4); 7,262 (8,6); 6,766 (0,7); 6,744 (0,7); 5,298 (0,7); 4,024 (6,4); 2,852 (16,0); 2,517 (7,5); 2,131 (12,3) |
| 1-455 | Me | SMe | c-Pr | H | 7.38 (d, 2H), 6.60 (d, 2H), 4.04 (s, 3H), 2.75-2.65 (m, 2H), 2.53 (s, 6H), 2.14 (s, 6H), 1.11-1.06 (m, 4H), 0.70-0.67 (m, 4H) |
| 1-456 | Me | SMe | OMe | H | ¹H-NMR (400,1 MHz, CDCl₃): 7,548 (1,6); 7,526 (1,7); 7,262 (11,5); 6,699 (1,3); 6,678 (1,2); 4,029 (7,8); 3,903 (11,1); 2,542 (9,1); 2,203 (16,0) |
| 1-457 | Me | SMe | OEt | H | ¹H-NMR (400,1 MHz, CDCl₃): 7,506 (1,6); 7,484 (1,7); 7,266 (0,4); 7,2654 (0,5); 7,2645 (0,8); 7,261 (26,5); 7,256 (0,8); 7,2554 (0,6); 7,2546 (0,5); 7,254 (0,5); 7,253 (0,4); 7,252 (0,3); 6,665 (1,3); 6,643 (1,3); 5,299 (2,2); 4,132 (0,8); 4,124 (0,4); 4,114 (2,6); 4,108 |
| | | | | | (0,5); 4,097 (2,6); 4,079 (0,9); 4,033 (0,5); 4,027 (7,5); 3,995 (0,8); 3,788 (1,0); 2,838 (0,4); 2,511 (9,3); 2,494 (0,5); 2,322 (0,9); 2,245 (1,4); 2,240 (0,4); 2,229 (0,7); 2,228 (0,7); 2,223 (16,0); 2,193 (1,5); 2,153 (0,4); 1,526 (0,4); 1,518 (0,4); 1,509 (0,9); 1,503 (2,9); 1,494 (1,1); 1,485 (6,1); 1,477 (0,7); 1,468 (2,8); 1,258 (0,5); 1,256 (0,5); 0,000 (8,8) |
| 1-458 | Me | SMe | OCH₂CF₃ | H | ¹H-NMR (400,0 MHz, d₆-DMSO): 7,789 (1,8); 7,767 (1,9); 7,030 (1,4); 7,008 (1,3); 4,901 (0,6); 4,880 (1,7); 4,858 (1,8); 4,836 (0,6); 4,145 (7,6); 3,321 (125,9); 3,297 (4,7); 2,706 (1,1); 2,524 (0,7); 2,519 (1,0); 2,510 (14,8); 2,506 (32,2); 2,501 (45,2); 2,497 (31,5); 2,492 (13,9); 2,427 (9,2); 2,279 (2,2); 2,169 (16,0); 0,858 (0,5); 0,000 (5,0) |
| 1-459 | Me | F | SMe | H | |
| 1-460 | Me | SMe | H | Me | ¹H-NMR (400,1 MHz, CDCl₃): 7,525 (0,4); 7,517 (1,5); 7,309 (1,3); 7,273 (0,6); 7,272 (0,6); 7,271 (0,7); 7,258 (264,8); 7,251 (2,7); 7,250 (2,5); 7,249 (2,2); 7,2474 (1,7); 7,2466 (1,6); 7,246 (1,4); 7,245 (1,3); 7,244 (1,1); 7,2433 (1,1); 7,2425 (1,0); 7,242 (0,9); 7,241 (0,9); 7,240 (0,8); 7,2393 (0,7); 7,2385 (0,7); 7,238 (0,6); 7,237 (0,6); 7,235 (0,5); 7,234 (0,5); 7,233 (0,4); 7,232 (0,4); 7,229 (0,4); 7,177 (0,5); 7,024 (1,7); 6,994 (1,5); 6,945 (1,7); 4,072 (10,7); 2,574 (2,3); 2,462 (4,6); 2,372 (16,0); 2,359 (2,4); 2,358 (2,4); 2,305 (7,5); 2,304 (7,8); 2,155 (8,8); 1,529 (3,4); 1,258 (1,2); 0,051 (0,4); 0,011 (0,5); 0,008 (2,7); 0,000 (86,3) |
| 1-461 | Me | NO₂ | H | Br | |
| 1-462 | Et | SMe | Br | H | |
| 1-463 | Et | SEt | Cl | H | |
| 1-464 | Et | SOMe | CF₃ | H | |
| 1-465 | Et | SOMe | Cl | H | |
| 1-466 | Et | SEt | CF₃ | H | |
| 1-467 | Et | SOEt | CF₃ | H | |
| 1-468 | Et | SO₂Et | CF₃ | H | |
| 1-469 | OMe | CH₂OMe | Cl | H | |
| 1-470 | OMe | CH₂-2H-tetrazol-2-yl | Cl | H | 8.50 (s, 2H), 7.59 (d, 2H), 7.30 (d, 2H), 6.06 (s, 2H), 4.02 (s, 3H), 3.93 (s, 6H) |
| 1-471 | OEt | SMe | CF₃ | H | ¹H-NMR (400,0 MHz, CDCl₃): 7,616 (1,1); 7,615 (1,1); 7,595 (2,3); 7,559 (3,3); 7,539 (1,6); 7,260 (21,7); 5,298 (1,1); 4,267 (1,1); 4,249 (3,5); 4,231 (3,6); 4,214 (1,1); 4,119 (11,8); 2,307 (16,0); 1,495 (4,7); 1,477 (10,2); 1,459 (4,5); 0,000 (7,1) |
| 1-472 | OEt | SO₂Me | CF₃ | H | ¹H-NMR (400,0 MHz, CDCl₃): 7,897 (0,6); 7,876 (4,3); 7,870 (5,5); 7,849 (0,8); 7,520 (0,6); 7,294 (0,6); 7,268 (0,7); 7,266 (1,1); 7,261 (109,0); 6,997 (0,6); 4,237 (0,9); 4,220 (2,8); 4,202 (2,9); 4,185 (0,9); 4,154 (13,5); 3,191 (16,0); 1,566 (4,3); 1,548 (13,4); 1,531 (4,4); 1,284 (0,8); 1,259 (1,8); 0,880 (0,7); 0,008 (1,5); 0,000 (48,2); -0,009 (1,3) |
| 1-473 | Cl | S-nPr | CF₃ | H | |
| 1-474 | Cl | SOEt | CF₃ | H | |
| 1-475 | Cl | SO₂Et | CF₃ | H | |
| 1-476 | Cl | Vinyl | SMe | H | |
| 1-477 | Cl | CH₂OMe | OMe | H | ¹H-NMR (400,0 MHz, CDCl₃): 7,596 (2,2); 7,574 (2,3); 7,260 (50,1); 6,811 (1,7); 6,789 (1,7); 4,557 (6,1); 4,060 (7,2); 3,868 (11,6); 3,325 (16,0); 1,537 (1,9); 1,256 (0,5); 0,008 (0,6); 0,000 (18,8); -0,009 (0,6) |
| 1-478 | Cl | CH₂OMe | SMe | H | |
| 1-479 | Cl | C(O)-c-Pr | Me | H | 7.47 (d, 2H), 7.17 (d, 2H), 4.09 (s, 3H), 2.26 (s, 6H), 2.12-2.07 (m, 2H), 1.32-1.29 (m, 4H), 1.15-1.10 (m, 4H) |
| 1-480 | Cl | CH(OMe)-i-Pr | Cl | H | |
| 1-481 | Cl | CH₂O-N=CH₂ | Cl | H | |
| 1-482 | Cl | CH₂O-N=CHMe | Cl | H | |
| 1-483 | Cl | CH₂O-N=CMe₂ | Cl | H | |
| 1-484 | Cl | [Diethyl(oxido)-lambda⁶-sulfanyliden]amin o | Cl | H | |
| 1-485 | Cl | (4-Oxido-1,4lambda⁴-oxathian-4-yliden)amino | Cl | H | |
| 1-486 | Cl | 2-Methylpyridin-3-yl | Me | H | |
| 1-487 | Cl | 3-methyl-4,5-dihydro-1,2-oxazol-5-yl | SMe | H | ¹H-NMR (400,0 MHz, CDCl₃): 7,519 (2,0); 7,460 (1,8); 7,451 (1,9); 7,440 (2,8); 7,430 (2,7); 7,368 (2,1); 7,344 (2,4); 7,323 (1,6); 7,310 (0,7); 7,294 (3,5); 7,260 (390,8); 7,229 (0,6); 6,996 (2,1); 6,503 (0,8); 6,492 (0,8); 6,474 (1,7); 6,462 (1,6); 6,445 (0,8); 6,433 (0,8); 4,169 (7,9); 4,161 (7,9); 4,131 (0,7); 4,113 (0,6); 3,259 (2,4); 3,232 (2,1); 2,349 (15,7); 2,344 (15,3); 2,091 (16,0); 2,045 (2,5); 2,007 (2,1); 1,544 (10,2); 1,277 (0,7); 1,259 (1,6); 1,241 (0,8); 0,034 (1,2); 0,008 (3,9); 0,000 (123,9);-0,009 (3,5) |
| 1-488 | Cl | SEt | CF₃ | H | |
| 1-489 | Cl | Me | F | Me | 7.55 (d,2H), 4.18 (s,3H), 2.20 (d,6H), 2.16 (d,6H) |
| 1-490 | Cl | H | F | Me | ¹H-NMR (400,0 MHz, CDCl₃): 7,519 (2,0); 7,460 (1,8); 7,451 (1,9); 7,440 (2,8); 7,430 (2,7); 7,368 (2,1); 7,344 (2,4); 7,323 (1,6); 7,310 (0,7); 7,294 (3,5); 7,260 (390,8); 7,229 (0,6); 6,996 (2,1); 6,503 (0,8); 6,492 (0,8); 6,474 (1,7); 6,462 (1,6); 6,445 (0,8); 6,433 (0,8); 4,169 (7,9); 4,161 (7,9); 4,131 (0,7); 4,113 (0,6); 3,259 (2,4); 3,232 (2,1); 2,349 (15,7); 2,344 (15,3); 2,091 (16,0); 2,045 (2,5); 2,007 (2,1); 1,544 (10,2); 1,277 (0,7); 1,259 (1,6); 1,241 (0,8); 0,034 (1,2); 0,008 (3,9); 0,000 (123,9); - 0,009 (3,5) |
| 1-491 | Br | CH₂OMe | SO₂Me | H | |
| 1-492 | Br | F | CF₃ | H | |
| 1-493 | Br | 1 H-pyrazol-1-yl | Cl | H | |
| 1-494 | I | SMe | H | Me | |
| 1-495 | CH₂OMe | SMe | CF₃ | H | |
| 1-496 | CH₂OMe | SCH₂CH₂OMe | CF₃ | H | |
| 1-497 | c-Pr | SOMe | CF₃ | H | |
| 1-498 | c-Pr | SMe | Et | H | ¹H-NMR (400,0 MHz, CDCl₃): 7,260 (29,5); 7,230 (1,1); 7,210 (1,5); 7,104 (1,4); 7,084 (1,1); 4,025 (7,7); 2,960 (0,7); 2,941 (2,1); 2,922 (2,2); 2,904 (0,7); 2,322 (16,0); 2,149 (0,6); 1,535 (1,0); 1,398 (0,7); 1,224 (3,2); 1,206 (7,2); 1,187 (3,1); 1,158 (1,1); 1,154 (1,2); 1,143 (0,5); 1,137 (1,1); 1,133 (1,1); 0,949 (1,0); 0,945 (1,3); 0,934 (1,1); 0,000 (12,8) |
| 1-499 | SMe | Vinyl | Cl | H | |
| 1-500 | SMe | 3-methyl-4,5-dihydro-1,2-oxazol-5-yl | Cl | H | |
| 1-501 | SO₂Me | 1H-pyrazol-1-yl | Cl | H | 8.63-8.38 (breit; 2H); 8.27 (s breit, 2H), 8.09 (s breit, 2H), 7.89 (s breit, 2H), 6.58 (s breit, 2H), 4.17 (s, 3H), 3.31 (s, 6H); |
| 1-502 | SO₂Me | 1H-pyrazol-1-yl | CF₃ | H | 8.31-7.96 (breit; 4H); 7.88-7.65 (breit, 4H), 6.55 (s breit, 2H), 4.23 (s, 3H), 3.27 (s, 6H); |
| 1-503 | SO₂Me | 1H-pyrazol-1-yl | OMe | H | 8.49-7.89 (breit; 4H); 7.85-7.39 (breit, 4H), 6.55 (s, 2H), 4.15 (s, 3H), 3.84 (s, 6H); 3.29 (s, 6H). |
| 1-504 | SO₂Me | 1H-pyrazol-1-yl | Me | H | 8.09-7.73 (m; 8H); 6.58 (s, 2H), 4.16 (s, 3H), 3.31 (s, 6H); 3.08 (s, 6H). |
| 1-505 | SO₂Me | 1H-pyrazol-1-yl | 1H-pyrazol-1-yl | H | |

**Tabelle 2: Erfindungsgemäße Verbindungen der Formel (I), worin Q für Q1, W für C-Y, R für substituiertes Phenyl und R⁶ für Ethyl steht. Tabelle 2 enthält 454 Verbindungen (2-1 bis 2-454), bei denen X, Y und Z wie in Beispielen 1-1 bis 1-454 der Tabelle 1 definiert sind, sowie nachfolgend genannte Beispiele:**

| | | | | | |
|---|---|---|---|---|---|
| 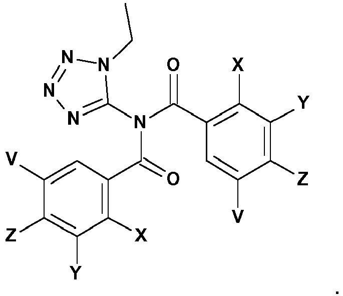 | | | | | |

| Nr. | X | Y | Z | V | Physikalische Daten (¹H-NMR, DMSO-d₆, 400 MHz) |
|---|---|---|---|---|---|
| 2-460 | Me | SMe | H | Me | 7.02 (s, 2H), 6.93 (s, 2H), 4.33 (q, 2H), 2.35 (s, 6H), 2.30 (s, 6H), 2.14 (s, 6H), 1.68 (t, 3H) |
| 2-461 | Me | NO₂ | H | Br | ¹H-NMR (400,0 MHz, CDCl₃): 8,292 (2,4); 8,287 (2,5); 8,106 (0,6); 8,101 (0,7); 8,039 (0,6); 8,034 (0,5); 8,017 (2,4); 8,012 (2,2); 7,259 (72,2); 4,553 (1,0); 4,535 (1,0); 3,501 (0,8); 2,610 (16,0); 2,576 (5,0); 1,684 (1,5); 1,666 (3,2); 1,647 (1,5); 0,008 (0,8); 0,000 (26,9); -0,009 (0,8) |
| 2-463 | Et | SEt | Cl | H | ¹H-NMR (400,0 MHz, CDCl₃): 7,393 (3,5); 7,372 (6,0); 7,317 (6,6); 7,296 (3,9); 7,260 (81,7); 4,309 (1,1); 4,291 (3,5); 4,273 (3,6); 4,254 (1,1); 2,988 (1,1); 2,969 (3,6); 2,951 (3,7); 2,932 (1,2); 2,830 (2,1); 2,811 (7,1); 2,793 (7,2); 2,774 (2,3); 2,044 (0,5); 1,689 (3,7); 1,671 (8,1); 1,653 (3,6); 1,551 (1,3); 1,259 (0,8); 1,256 (0,8); 1,234 (4,6); 1,216(11,1); 1,197 (5,4); 1,195 (8,9); 1,176 (16,0); 1,158 (7,3); 0,008 (0,8); 0,000 (26,8); -0,009 (0,9) |
| 2-477 | Cl | CH₂OMe | OMe | H | ¹H-NMR (400,0 MHz, CDCl₃): 7,607 (2,1); 7,586 (2,3); 7,260 (91,8); 6,996 (0,5); 6,799 (1,6); 6,777 (1,6); 5,298 (0,7); 4,552 (5,7); 4,342 (1,5); 4,323 (1,5); 3,863 (11,0); 3,322 (16,0); 2,101 (2,1); 1,649 (1,6); 1,631 (3,5); 1,613 (1,6); 1,536 (1,5); 1,256 (0,8); 0,008 (1,1); 0,000 (35,0); -0,009 (1,0) |
| 2-480 | Cl | CH(OMe)-i-Pr | Cl | H | ¹H-NMR (400,0 MHz, CDCl₃): 7,519 (1,0); 7,409 (3,1); 7,389 (9,0); 7,367 (5,5); 7,346 (2,0); 7,260 (190,6); 7,253 (0,8); 6,996 (1,0); 4,594 (1,3); 4,348 (1,4); 4,331 (1,4); 3,137 (6,9); 2,495 (0,6); 2,005 (0,5); 1,690 (7,1); 1,671 (15,7); 1,653 (7,1); 1,256 (0,8); 1,149 (16,0); 1,132 (15,5); 0,655 (5,0); 0,639 (5,2); 0,008 (2,3); 0,007 (0,8); 0,006 (0,9); 0,005 (1,0); 0,004 (1,4); 0,000 (66,8); -0,005 (0,7); -0,006 (0,5); -0,008 (1,7) |
| 2-499 | SMe | Vinyl | Cl | H | ¹H-NMR (400,0 MHz, CDCl₃): 7,402 (1,7); 7,381 (2,3); 7,266 (2,0); 7,260 (17,6); 7,246 (1,4); 6,846 (0,8); 6,815 (0,6); 6,803 (0,6); 6,772 (0,9); 5,754 (5,2); 5,727 (1,4); 5,724 (1,8); 5,711 (1,7); 5,708 (1,3); 4,519 (0,5); 4,500 (1,7); 4,482 (1,7); 4,464 (0,5); 2,274 (16,0); 1,672 (1,8); 1,654 (4,0); 1,636 (1,8); 1,542 (1,6); 0,000 (6,1) |
| 2-500 | SMe | 3-methyl-4,5-dihydro-1,2-oxazol-5-yl | Cl | H | ¹H-NMR (400,0 MHz, CDCl₃): 7,445 (1,7); 7,434 (1,9); 7,425 (2,7); 7,413 (2,7); 7,361 (1,7); 7,340 (1,2); 7,334 (1,8); 7,313 (1,3); 7,261 (76,7); 7,228 (0,7); 6,504 (0,8); 6,488 (0,8); 6,475 (1,7); 6,459 (1,5); 6,446 (0,8); 6,430 (0,8); 4,484 (1,1); 4,470 (1,7); 4,466 (1,3); 4,451 (1,6); 3,260 (2,1); 3,258 (2,1); 3,235 (1,7); 3,227 (1,9); 2,342 (15,5); 2,336 (14,9); 2,092 (16,0); 2,045 (0,8); 1,674 (1,9); 1,670 (2,0); 1,656 (4,4); 1,652 (4,4); 1,637 (2,0); 1,633 (2,0); 1,259 (0,6); 0,008 (0,7); 0,000 (22,1); -0,009 (0,6) |

**Tabelle 3: Erfindungsgemäße Verbindungen der Formel (I), worin Q für Q2, W für C-Y, R für substituiertes Phenyl und R⁷ für Methyl steht. Tabelle 3 enthält 454 Verbindungen (3-1 bis 3-454), bei denen X, Y und Z wie in Beispielen 1-1 bis 1-454 der Tabelle 1 definiert sind, sowie nachfolgend genannte Beispiele:**

| | | | | | |
|---|---|---|---|---|---|
| 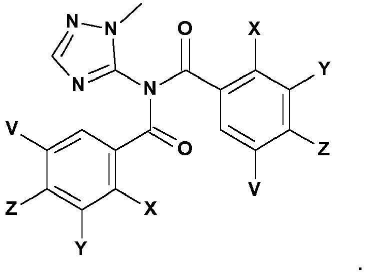 | | | | | |

| Nr. | X | Y | Z | V | Physikalische Daten (¹H-NMR, DMSO-d₆, 400 MHz) |
|---|---|---|---|---|---|
| 3-95 | Me | F | SMe | H | 7.95 (s,1H), 7.63 (d,2H), 7.19 (dd,2H), 3.86 (s,3H), 2.50 (s,6H), 2.24 (d,6H) |
| 3-143 | Me | SMe | CF₃ | H | 7.99 (s,1H), 7.94 (d,2H), 7.71 (d,2H), 3.95 (s,3H), 2.64 (s,6H), 2.22 (d,6H) |
| 3-146 | Me | SEt | CF₃ | H | ¹H-NMR (400,1 MHz, CDCl₃): 7,757 (4,6); 7,602 (1,7); 7,582 (2,5); 7,520 (0,4); 7,489 (2,2); 7,469 (1,6); 7,272 (0,3); 7,271 (0,4); 7,270 (0,5); 7,269 (0,7); 7,2683 (0,8); 7,2675 (1,0); 7,267 (1,2); 7,261 (75,4); 7,2534 (0,8); 7,2526 (0,7); 7,252 (0,7); 7,251 (0,6); 7,250 (0,5); 7,249 (0,5); 7,248 (0,4); 7,247 (0,4); 7,246 (0,3); 6,998 (0,4); 3,868 (13,3); 2,899 (1,4); 2,757 (0,4); 2,738 (16,0); 2,716 (1,3); 2,697 (3,7); 2,679 (3,8); 2,660 (1,3); 1,561 (1,0); 1,233 (0,5); 1,215 (1,0); 1,201 (5,0); 1,182 (10,3); 1,164 (4,8); 0,008 (0,8); 0,006 (0,4); 0,005 (0,5); 0,004 (0,7) |
| 3-202 | Et | SMe | CF₃ | H | |
| 3-470 | OMe | CH₂-2H-tetrazol-2-yl | Cl | H | 8.49 (s, 2H), 7.76 (s, 1H), 7.58 (d, 2H), 7.28 (d, 2H), 5.91 (s, 2H), 3.93 (s, 6H), 3.79 (s, 3H) |
| 3-475 | Cl | SO₂Et | CF₃ | H | ¹H-NMR (400,0 MHz, CDCl₃): 7,985 (2,4); 7,964 (3,0); 7,797 (2,2); 7,776 (1,9); 7,744 (5,8); 7,267 (0,8); 7,261 (71,7); 4,148 (0,6); 4,131 (1,7); 4,113 (1,7); 4,095 (0,6); 3,940 (16,0); 3,923 (0,7); 3,867 (0,7); 3,835 (0,6); 3,500 (1,7); 3,481 (5,6); 3,462 (5,7); 3,444 (1,8); 2,097 (0,6); 2,045 (8,1); 1,455 (0,5); 1,450 (0,5); 1,438 (5,8); 1,419 (12,6); 1,401 (5,5); 1,277 (2,6); 1,266 (1,0); 1,259 (5,3); 1,241 (2,4); 0,882 (1,7); 0,864 (0,6); 0,008 (1,0); 0,000 (30,6); - 0,009 (0,9) |
| 3-476 | Cl | Vinyl | SMe | H | ¹H-NMR (400,0 MHz, CDCl₃): 7,876 (3,7); 7,434 (2,2); 7,433 (2,2); 7,413 (2,3); 7,412 (2,4); 7,261 (24,0); 6,999 (2,4); 6,978 (2,2); 6,519 (1,1); 6,490 (1,2); 6,474 (1,2); 6,446 (1,3); 5,737 (2,4); 5,734 (2,5); 5,708 (2,3); 5,705 (2,4); 5,564 (2,5); 5,561 (2,4); 5,519 (2,3); 5,516 (2,2); 3,908 (12,0); 2,416 (16,0); 1,255 (0,8); 0,000 (7,3) |
| 3-477 | Cl | CH₂OMe | OMe | H | ¹H-NMR (400,0 MHz, CDCl₃): 7,804 (2,4); 7,602 (2,0); 7,581 (2,1); 7,268 (0,5); 7,2674 (0,6); 7,2666 (0,7); 7,266 (0,8); 7,265 (1,0); 7,259 (75,8); 7,255 (0,8); 7,254 (0,5); 6,802 (1,4); 6,780 (1,4); 5,298 (0,9); 4,576 (5,0); 3,873 (6,8); 3,865 (10,0); 3,325 (16,0); 1,536 (7,0); 0,008 (0,9); 0,000 (30,4); -0,009 (0,9) |
| 3-479 | Cl | C(O)-c-Pr | Me | H | 7.84 (s, 1H), 7.47 (d, 2H), 7.15 (d, 2H), 3.89 (s, 3H), 3.25 (s, 6H), 2.14-2.10 (m, 2H), 1.32-1.28 (m, 4H), 1.14-1.09 (m, 4H) |
| 3-480 | Cl | CH(OMe)-i-Pr | Cl | H | Beispiel 3-480: ¹H-NMR (400,0 MHz, CDCl₃): 7,689 (4,9); 7,519 (0,7); 7,385 (1,6); 7,365 (3,9); 7,350 (0,6); 7,340 (4,9); 7,319 (2,0); 7,260 (127,0); 6,996 (0,7); 4,634 (1,0); 4,612 (1,1); 3,905 (7,4); 3,154 (13,8); 2,534 (0,6); 1,154 (16,0); 1,138 (15,6); 0,659 (3,7); 0,646 (3,9); 0,008 (1,3); 0,000 (44,3); - 0,009 (1,3) |
| 3-486 | Cl | 2-Methylpyridin-3-yl | Me | H | Beispiel 3-486: ¹H-NMR (400,0 MHz, CDCl₃): 8,606 (1,7); 8,593 (1,7); 7,764 (3,7); 7,756 (3,3); 7,519 (0,9); 7,514 (2,2); 7,509 (1,8); 7,494 (2,6); 7,489 (2,2); 7,350 (1,4); 7,334 (1,9); 7,330 (1,9); 7,311 (0,6); 7,284 (3,4); 7,274 (2,3); 7,261 (125,8); 7,244 (1,1); 6,997 (0,7); 3,939 (2,5); 3,908 (1,1); 3,873 (14,3); 2,320 (1,4); 2,298 (1,8); 2,271 (0,6); 2,218 (7,8); 2,206 (8,0); 2,140 (0,6); 2,089 (0,5); 2,061 (1,1); 2,038 (1,9); 2,032 (1,6); 2,013 (16,0); 0,008 (1,6); 0,000 (45,1); - 0,009 (1,1) |
| 3-487 | Cl | 3-methyl-4,5-dihydro-1,2-oxazol-5-yl | SMe | H | ¹H-NMR (400,0 MHz, CDCl₃): 7,926 (3,8); 7,912 (3,8); 7,522 (0,6); 7,463 (3,0); 7,453 (2,9); 7,442 (3,5); 7,433 (3,2); 7,273 (0,5); 7,272 (0,6); 7,269 (1,0); 7,263 (115,7); 7,104 (3,5); 7,083 (3,1); 6,999 (0,6); 6,152 (1,0); 6,141 (1,0); 6,123 (2,4); 6,112 (2,5); 6,093 (1,0); 6,082 (1,0); 4,131 (0,8); 4,113 (0,8); 3,954 (1,0); 3,928 (0,6); 3,922 (10,8); 3,919 (10,7); 3,910 (0,5); 3,181 (0,9); 3,178 (1,0); 3,172 (1,1); 3,170 (1,1); 3,162 (1,1); 3,160 (1,1); 3,149 (1,8); 3,143 (1,2); 3,140 (1,1); 3,132 (1,1); 3,130 (1,0); 3,121 (1,0); 3,119 (1,1); 2,483 (15,7); 2,481 (16,0); 2,473 (1,6); 2,468 (0,8); 2,465 (0,9); 2,348 (0,5); 2,336 (0,5); 2,094 (1,7); 2,075 (12,2); 2,072 (12,0); 2,045 (3,9); 1,583 (1,1); 1,333 (0,5); 1,284 (0,8); 1,277 (1,3); 1,259 (4,1); 1,243 (1,4); 1,241 (1,3); 1,226 (0,7); 0,880 (0,7); 0,008 (0,6); 0,000 (24,6); - 0,009 (0,7) |
| 3-490 | Cl | H | F | Me | 7.99 (s,1H), 7.72 (d,2H), 7.47 (d,2H), 3.90 (s,3H), 2.23 (s,6H) |
| 3-491 | Br | CH₂OMe | SO₂Me | H | |
| 3-492 | Br | F | CF₃ | H | ¹H-NMR (400,0 MHz, CDCl₃): 7,775 (1,7); 7,428 (0,5); 7,2674 (0,5); 7,2666 (0,6); 7,266 (0,7); 7,265 (0,9); 7,264 (1,2); 7,260 (71,3); 7,255 (0,5); 3,957 (5,7); 1,539 (16,0); 0,008 (0,8); 0,000 (27,8); -0,009 (0,8) |
| 3-493 | Br | 1H-pyrazol-1-yl | Cl | H | 7.84 (d,2H), 7.81 (s,1H), 7.60 (d,2H), 7.56 (d, 2H), 7.53 (d, 2H), 6.53 (t, 2H), 3.91 (s,3H), |
| 3-494 | I | SMe | H | Me | ¹H-NMR (400, 1 MHz, CDCl₃): 7,881 (3,1); 7,518 (1,2); 7,309 (0,5); 7,2803 (0,3); 7,2795 (0,4); 7,279 (0,4); 7,278 (0,4); 7,277 (0,4); 7,2763 (0,5); 7,2755 (0,5); 7,275 (0,5); 7,274 (0,6); 7,273 (0,6); 7,272 (0,7); 7,2714 (0,8); 7,2705 (0,9); 7,270 (1,0); 7,269 (1,1); 7,268 (1,3); 7,2673 (1,4); 7,2665 (1,8); 7,266 (2,1); 7,265 (2,8); 7,264 (3,6); 7,263 (4,7); 7,260 (208,2); 7,254 (4,4); 7,253 (3,7); 7,2523 (3,1); 7,2515 (2,6); 7,251 (2,2); 7,250 (2,0); 7,249 (1,6); 7,248 (1,5); 7,2474 (1,3); 7,2466 (1,2); 7,246 (1,0); 7,245 (1,0); 7,244 (0,9); 7,2433 (0,8); 7,2425 (0,7); 7,242 (0,7); 7,241 (0,6); 7,240 (0,6); 7,239 (0,5); 7,2384 (0,4); 7,2376 (0,4); 7,237 (0,4); 7,236 (0,4); 7,235 (0,4); 7,2343 (0,4); 7,2336 (0,4); 7,104 (1,7); 7,101 (1,8); 6,996 (1,2); 6,824 (1,7); 6,820 (1,7); 4,113 (0,3); 3,973 (10,2); 3,887 (0,4); 2,435 (0,4); 2,425 (0,5); 2,408 (16,0); 2,332 (11,1); 2,043 (1,6); 1,540 (91,4); 1,276 (0,6); 1,264 (0,4); 1,258 (1,1); 1,240 (0,5); 0,882 (0,7); 0,008 (1,9) |
| 3-495 | CH₂OMe | SMe | CF₃ | H | ¹H-NMR (400,0 MHz, CDCl₃): 7,722 (2,6); 7,681 (1,2); 7,660 (2,0); 7,606 (1,5); 7,585 (0,9); 7,261 (24,0); 5,299 (1,1); 5,114 (5,9); 3,849 (8,0); 3,369 (16,0); 2,251 (12,5); 1,371 (1,1); 1,333 (1,2); 1,285 (2,3); 1,256 (2,9); 0,000 (9,1) |
| 3-500 | SMe | 3-methyl-4,5-dihydro-1,2-oxazol-5-yl | Cl | H | ¹H-NMR (400,0 MHz, CDCl₃): 7,775 (2,6); 7,759 (2,3); 7,520 (0,5); 7,438 (1,9); 7,432 (1,7); 7,418 (3,1); 7,411 (2,5); 7,359 (2,0); 7,339 (2,4); 7,319 (1,0); 7,261 (90,4); 6,542 (0,7); 6,531 (0,8); 6,513 (1,8); 6,501 (1,5); 6,484 (0,8); 6,473 (0,8); 3,971 (7,1); 3,966 (8,5); 3,268 (2,1); 3,239 (1,9); 3,229 (1,2); 2,458 (1,0); 2,348 (12,9); 2,342 (14,9); 2,133 (0,9); 2,101 (1,1); 2,089 (16,0); 2,045 (0,7); 1,560 (1,0); 1,259 (0,8); 0,008 (0,9); 0,000 (26,7); -0,009 (0,7) |

**Tabelle 4: Erfindungsgemäße Verbindungen der Formel (I), worin Q für Q3, W für C-Y, R für substituiertes Phenyl und R⁸ für Methyl steht. Tabelle 4 enthält 454 Verbindungen (4-1 bis 4-454), bei denen X, Y und Z wie in Beispielen 1-1 bis 1-454 der Tabelle 1 definiert sind.**

| | | | | | |
|---|---|---|---|---|---|
| 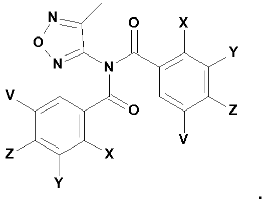 | | | | | |

| Nr. | X | Y | Z | V | Physikalische Daten (¹H-NMR, DMSO-d₆, 400 MHz) |
|---|---|---|---|---|---|
| 4-35 | NO₂ | H | F | H | 8.26 (dd,2H), 7.99 (dd,2H), 7.88 (dd, 2H), 1.52 (s,3H) |
| 4-36 | NO₂ | H | Cl | H | 8.22 (dd,2H), 7.73 (dd,2H), 7.49 (d, 2H), 2.48 (s,3H) |
| 4-144 | Me | SOMe | CF₃ | H | 7.61 (2d,2H), 7.49 (2d,2H), 2.96 (s,6H), 2.93 (s,6H), 2.41 (s,3H) |
| 4-157 | Me | NH₂ | SO₂Me | H | 7.45 (d,2H), 6.98 (d,2H), 5.99 (s, 4H), 3.10 (s,6H), 2.45 (s,3H), 1.99 (s,6H) |
| 4-185 | Me | SO₂Me | SO₂Me | H | 8.37 (d,2H), 8.18 (d,2H), 3.56 (s,6H), 3.51 (s,6H), 2.69 (s,3H) |
| 4-187 | Me | SEt | SO₂Me | H | ¹H-NMR (400,1 MHz, CDCl₃): 8,121 (2,2); 8,101 (2,4); 7,520 (0,7); 7,518 (0,7); 7,464 (2,6); 7,443 (2,5); 7,268 (0,5); 7,267 (0,6); 7,261 (114,8); 7,259 (126,6); 7,251 (1,3); 7,250 (1,1); 7,249 (1,0); 7,2484 (0,9); 7,2476 (0,8); 7,247 (0,7); 7,246 (0,6); 7,245 (0,5); 7,2443 (0,5); 7,2435 (0,5); 7,243 (0,4); 7,242 (0,4); 7,241 (0,4); 7,240 (0,4); 7,239 (0,3); 6,997 (0,7); 6,996 (0,7); 5,298 (0,7); 3,506 (0,5); 3,486 (0,5); 3,436 (16,0); 3,047 (0,3); 2,921 (0,3); 2,911 (1,2); 2,893 (3,8); 2,874 (3,9); 2,856 (1,3); 2,739 (13,3); 2,628 (0,4); 2,433 (7,9); 2,432 (8,1); 1,532 (44,2); 1,531 (47,6); 1,312 (0,4); 1,280 (4,1); 1,261 (8,3); 1,243 (3,9); 0,010 (1,3); 0,008 (1,6); 0,001 (47,4); 0,000 (52,7) |
| 4-188 | Me | SOEt | SO₂Me | H | ¹H-NMR (400,1 MHz, CDCl₃): 8,105 (1,8); 8,085 (2,0); 8,045 (1,3); 8,025 (1,5); 7,584 (2,3); 7,564 (2,1); 7,414 (2,3); 7,394 (2,1); 7,261 (31,7); 3,442 (16,0); 3,418 (0,4); 3,399 (0,3); 3,395 (0,4); 3,338 (12,4); 3,242 (0,7); 3,223 (0,8); 3,210 (0,6); 3,190 (0,6); 2,908 (6,3); 2,890 (1,9); 2,879 (1,7); 2,871 (1,7); 2,861 (1,6); 2,852 (0,7); 2,842 (0,5); 2,832 (0,4); 2,726 (11,6); 2,437 (15,9); 2,043 (1,3); 1,552 (10,7); 1,537 (3,2); 1,518 (6,6); 1,499 (3,0); 1,273 (4,0); 1,254 (8,5); 1,236 (3,7); 0,008 (0,5) |
| 4-199 | CH₂O(CH₂)₂O Me | NH(CH₂)₃OMe | SO₂Me | H | 7.74 (d,2H), 7.12 (d,2H), 4.63 (s, 4H), 3.59-3.47 (m, 12H), 3.39 (s, 6H), 3.35 (s, 6H), 3.26 (t, 4H), 3.09 (s, 6H), 2.45 (s, 3H), 1.94-1.87 (m, 4H) |
| 4-201 | Et | SO₂Me | Cl | H | ¹H-NMR (400,1 MHz, CDCl₃): 7,494 (2,1); 7,474 (3,3); 7,406 (3,0); 7,385 (1,9); 7,262 (8,9); 5,299 (5,8); 3,352 (0,6); 3,342 (16,0); 3,263 (0,6); 3,244 (1,7); 3,226 (1,7); 3,208 (0,6); 2,413 (8,1); 2,043 (1,3); 1,550 (2,5); 1,325 (2,7); 1,307 (6,2); 1,288 (2,7); 1,276 (0,5); 1,258 (0,8); 1,240 (0,4); 0,000 (3,8) |
| 4-205 | Et | NH(CH₂)₂OMe | SO₂Me | H | 7.79 (d, 2H), 6.96 (d, 2H), 3.60 (t, 4H), 3.40 (s, 6H), 3.38 (t, 4H), 3.20 (s, 6H), 2.75 (q, 4H), 2.40 (s, 3H), 1.21 (t, 6H) |
| 4-358 | SMe | SMe | F | H | 7.30 (dd, 2H), 7.07 (dd, 2H), 2.50 (s, 6H), 2.48 (s, 3H), 2.39 (s, 6H) |
| 4-430 | Me | CH₂SMe | CF₃ | H | ¹H-NMR (400,0 MHz, CDCl₃): 7,506 (1,6); 7,485 (2,0); 7,337 (1,8); 7,317 (1,4); 7,265 (0,6); 7,264 (0,8); 7,263 (1,0); 7,260 (45,6); 3,805 (5,7); 2,608 (0,6); 2,540 (14,0); 2,424 (14,9); 2,387 (0,6); 2,189 (0,5); 2,163 (0,8); 2,115 (16,0); 1,535 (5,3); 0,008 (0,5); 0,000 (18,7) |
| 4-448 | Me | NMe₂ | CF₃ | H | ¹H-NMR (400,0 MHz, CDCl₃): 7,454 (1,0); 7,433 (1,2); 7,301 (1,1); 7,281 (0,9); 7,260 (35,3); 2,692 (16,0); 2,430 (8,0); 2,287 (8,3); 1,539 (14,4); 0,000 (12,0) |
| 4-449 | Me | NHMe | CF₃ | H | ¹H-NMR (400,0 MHz, CDCl₃): 7,364 (2,0); 7,343 (2,2); 7,260 (43,3); 7,067 (1,9); 7,047 (1,7); 2,736 (13,1); 2,421 (14,5); 2,352 (16,0); 1,543 (0,8); 1,255 (1,0); 0,000 (14,9) |
| 4-451 | Me | SMe | Me | H | ¹H-NMR (400,0 MHz, CDCl₃): 7,302 (1,4); 7,283 (1,7); 7,260 (14,1); 7,073 (1,2); 7,053 (1,0); 2,554 (8,7); 2,497 (8,1); 2,404 (8,4); 2,243 (0,7); 2,090 (16,0); 1,546 (1,7); 1,366 (0,8); 0,000 (4,8) |
| 4-452 | Me | SEt | OMe | H | 7.48 (d, 2H), 6.66 (d, 2H), 3.88 (s, 6H), 2.69 (q, 4H), 2.51(s, 6H), 2.39 (s, 3H), 1.01 (t, 6H) |
| 4-457 | Me | SMe | OEt | Me | ¹H-NMR (400,1 MHz, CDCl₃): 7,396 (1,7); 7,374 (1,8); 7,265 (0,4); 7,260 (16,4); 6,647 (1,3); 6,625 (1,3); 5,298 (1,1); 4,125 (0,8); 4,107 (2,5); 4,090 (2,6); 4,072 (0,8); 2,696 (0,8); 2,522 (0,4); 2,505 (0,4); 2,495 (9,4); 2,481 (1,3); 2,396 (1,5); 2,392 (7,8); 2,383 (0,4); 2,357 (1,4); 2,325 (0,5); 2,248 (0,5); 2,231 (0,7); 2,228 (0,4); 2,221 (16,0); 1,528 (0,6); 1,510 (0,4); 1,500 (2,9); 1,492 (0,5); 1,482 (6,3); 1,465 (2,8) |
| 4-460 | Me | SMe | H | Me | |
| 4-466 | Et | SEt | CF₃ | H | ¹H-NMR (400,0 MHz, CDCl₃): 7,607 (2,9); 7,586 (3,5); 7,392 (2,4); 7,372 (2,0); 7,260 (39,8); 3,143 (1,0); 3,124 (3,5); 3,106 (3,6); 3,087 (1,1); 2,745 (1,4); 2,726 (4,5); 2,708 (4,6); 2,689 (1,5); 2,415 (16,0); 1,538 (14,0); 1,292 (4,5); 1,273 (10,9); 1,255 (4,9); 1,251 (6,6); 1,232 (12,3); 1,213 (5,6); 0,008 (0,5); 0,000 (17,2); -0,009 (0,5) |
| 4-469 | OMe | CH₂OMe | Cl | H | 7.42 (d, 2H), 7.35 (d, 2H), 4.38 (s, 4H), 3.92 (s, 6H), 3.40 (s, 6H), 2.42 (s, 3H) |
| 4-470 | OMe | CH₂-2H-tetrazol-2-yl | Cl | H | 8.49 (s, 2H), 7.53 (d, 2H), 7.26 (d, 2H), 5.85 (s, 4H), 3.93 (s, 6H), 2.39 (s, 3H) |
| 4-471 | OEt | SMe | CF₃ | H | 7.51 (d, 2H), 7.49 (d, 2H), 4.22 (q, 4H), 2.44 (s, 3H), 2.30 (s, 6H), 1.46 (t, 6H) |
| 4-477 | Cl | CH₂OMe | OMe | H | ¹H-NMR (400,0 MHz, CDCl₃): 7,540 (2,1); 7,519 (2,4); 7,260 (30,9); 6,805 (1,5); 6,783 (1,4); 4,565 (5,4); 3,866 (10,5); 3,326 (16,0); 2,419 (7,1); 1,537 (1,2); 0,000 (11,9) |
| 4-481 | Cl | CH₂O-N=CH₂ | Cl | H | |
| 4-482 | Cl | CH₂O-N=CHMe | Cl | H | |
| 4-483 | Cl | CH₂O-N=CMe₂ | Cl | H | |
| 4-487 | Cl | 3-methyl-4,5-dihydro-1,2-oxazol-5-yl | SMe | H | ¹H-NMR (400,0 MHz, CDCl₃): 7,520 (0,7); 7,414 (2,6); 7,401 (2,3); 7,393 (3,0); 7,380 (2,6); 7,273 (0,5); 7,2724 (0,5); 7,2716 (0,6); 7,271 (0,7); 7,270 (0,7); 7,269 (0,9); 7,2684 (1,1); 7,2676 (1,2); 7,267 (1,4); 7,266 (1,7); 7,265 (2,3); 7,261 (126,8); 7,083 (3,0); 7,063 (2,6); 6,997 (0,7); 6,137 (0,8); 6,129 (0,9); 6,108 (2,0); 6,099 (2,1); 6,078 (0,9); 6,070 (0,9); 4,149 (1,1); 4,131 (3,3); 4,113 (3,4); 4,095 (1,1); 3,175 (0,8); 3,166 (0,8); 3,159 (0,9); 3,146 (1,5); 3,139 (0,9); 3,137 (0,9); 3,130 (0,9); 3,128 (0,8); 3,118 (0,9); 2,485 (14,4); 2,476 (1,1); 2,472 (1,0); 2,469 (1,2); 2,443 (15,8); 2,319 (0,6); 2,095 (0,7); 2,080 (10,1); 2,077 (9,8); 2,045 (16,0); 1,548 (45,2); 1,277 (4,8); 1,259 (10,1); 1,241 (4,7); 0,008 (1,6); 0,006 (0,6); 0,005 (0,7); 0,004 (0,9); 0,000 (49,9); - 0,005 (0,7); -0,009 (1,3) |
| 4-495 | CH₂OMe | SMe | CF₃ | H | ¹H-NMR (400,1 MHz, CDCl₃): 7,664 (1,2); 7,644 (1,6); 7,500 (1,3); 7,480 (1,0); 7,259 (17,1); 5,017 (5,7); 3,405 (0,4); 3,392 (16,0); 3,353 (0,4); 2,593 (0,4); 2,413 (7,7); 2,270 (0,5); 2,269 (0,4); 2,255 (11,1); 2,043 (0,6); 1,569 (0,4); 1,432 (3,8); 1,258 (0,4); 0,000 (7,2) |
| 4-496 | CH₂OMe | SCH₂CH₂OMe | CF₃ | H | |

**Tabelle 5: Erfindungsgemäße Verbindungen der Formel (I), worin Q für Q3, W für C-Y, W für C-Y, R für substituiertes Phenyl und R⁸ für Chlor steht. Tabelle 5 enthält 454 Verbindungen (5-1 bis 5-454), bei denen X, Y und Z wie in Beispielen 1-1 bis 1-454 der Tabelle 1 definiert sind, sowie nachfolgend genannte Beispiele.**

| | | | | | |
|---|---|---|---|---|---|
| 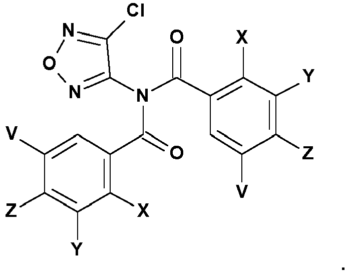 | | | | | |

| Nr. | X | Y | Z | V | Physikalische Daten (¹H-NMR, DMSO-d₆, 400 MHz) |
|---|---|---|---|---|---|
| 5-451 | Me | SMe | Me | H | ¹H-NMR (400,0 MHz, CDCl₃): 7,350 (1,3); 7,330 (1,5); 7,260 (36,4); 7,087 (1,1); 7,067 (0,9); 2,572 (8,2); 2,505 (7,6); 2,099 (16,0); 1,536 (12,0); 0,000 (13,0) |
| 5-462 | Et | SMe | Br | H | |
| 5-466 | Et | SEt | CF₃ | H | ¹H-NMR (400,0 MHz, CDCl₃): 7,628 (3,9); 7,607 (4,8); 7,519 (2,2); 7,469 (3,8); 7,448 (2,9); 7,293 (2,3); 7,260 (412,7); 7,225 (0,8); 6,996 (2,2); 3,152 (1,7); 3,133 (4,9); 3,115 (5,1); 3,096 (1,7); 2,748 (2,1); 2,729 (6,2); 2,710 (6,4); 2,692 (2,1); 1,570 (1,0); 1,537 (164,7); 1,293 (6,9); 1,275 (15,5); 1,256 (8,3); 1,252 (9,0); 1,233 (16,0); 1,214 (7,3); 0,899 (1,4); 0,882 (3,9); 0,864 (1,6); 0,146 (0,7); 0,033 (1,0); 0,008 (8,6); 0,000 (170,3); -0,009 (4,5); -0,150 (0,7) |
| 5-477 | Cl | CH₂OMe | OMe | H | ¹H-NMR (400,0 MHz, CDCl₃): 7,587 (2,0); 7,565 (2,2); 7,268 (0,5); 7,2673 (0,6); 7,2665 (0,7); 7,266 (0,9); 7,265 (1,0); 7,264 (1,3); 7,263 (1,6); 7,259 (89,9); 7,256 (1,8); 7,255 (1,2); 7,2543 (0,8); 7,2535 (0,6); 6,816 (1,4); 6,794 (1,3); 4,565 (5,0); 3,873 (9,8); 3,324 (16,0); 1,531 (10,7); 0,008 (1,1); 0,000 (36,7); -0,009 (1,0) |
| 5-484 | Cl | [Diethyl(oxido)-lambda⁶-sulfanyliden]amino | Cl | H | ¹H-NMR (400,0 MHz, CDCl₃): 7,521 (0,6); 7,330 (20,6); 7,320 (0,6); 7,319 (0,6); 7,309 (26,0); 7,270 (0,7); 7,262 (100,4); 7,140 (24,3); 7,134 (0,5); 7,119 (20,6); 6,998 (0,6); 4,130 (1,3); 4,113 (1,3); 3,281 (0,7); 3,263 (2,2); 3,246 (5,4); 3,235 (4,9); 3,227 (15,9); 3,217 (15,5); 3,209 (16,0); 3,198 (16,0); 3,190 (5,3); 3,180 (5,7); 3,163 (2,2); 3,145 (0,7); 2,045 (6,0); 1,555 (20,5); 1,478 (43,4); 1,460 (97,5); 1,441 (41,6); 1,425 (0,7); 1,295 (0,6); 1,277 (2,1); 1,259 (4,5); 1,247 (0,5); 1,241 (1,9); 0,008 (1,4); 0,000 (48,1); -0,009 (1,3) |
| 5-485 | Cl | (4-Oxido-1,4lambda⁴-oxathian-4-yliden)amino | Cl | H | ¹H-NMR (400,0 MHz, CDCl₃): 7,713 (0,7); 7,692 (0,7); 7,529 (0,8); 7,520 (3,7); 7,508 (0,9); 7,492 (0,6); 7,482 (1,3); 7,420 (0,6); 7,390 (1,5); 7,369 (1,0); 7,354 (1,3); 7,349 (1,8); 7,343 (47,9); 7,333 (1,4); 7,328 (2,1); 7,322 (57,2); 7,302 (0,5); 7,298 (0,6); 7,295 (0,6); 7,291 (0,9); 7,290 (0,9); 7,289 (0,8); 7,2864 (0,9); 7,2856 (1,0); 7,285 (1,0); 7,284 (1,1); 7,283 (1,2); 7,2824 (1,3); 7,2816 (1,3); 7,281 (1,4); 7,280 (1,4); 7,279 (1,6); 7,2784 (1,7); 7,2776 (1,9); 7,277 (2,0); 7,276 (2,2); 7,2752 (2,5); 7,2745 (2,5); 7,274 (2,8); 7,273 (3,1); 7,272 (3,4); 7,271 (3,4); 7,2704 (3,8); 7,2696 (4,6); 7,269 (5,7); 7,268 (5,8); 7,2673 (6,7); 7,2665 (8,2); 7,266 (9,8); 7,265 (12,3); 7,264 (17,0); 7,261 (625,8); 7,256 (6,1); 7,255 (3,7); 7,254 (2,7); 7,2534 (2,4); 7,2526 (1,8); 7,252 (1,5); 7,251 (1,4); 7,250 (1,1); 7,2494 (0,9); 7,2486 (0,9); 7,248 (1,2); 7,247 (0,7); 7,228 (0,6); 7,211 (0,5); 7,173 (0,8); 7,160 (55,2); 7,139 (45,5); 7,126 (0,6); 6,997 (3,6); 4,328 (6,6); 4,320 (8,8); 4,314 (7,6); 4,305 (7,3); 4,297 (10,0); 4,288 (11,1); 4,282 (13,5); 4,273 (10,5); 4,193 (11,4); 4,187 (13,2); 4,172 (12,2); 4,165 (13,1); 4,155 (8,3); 4,149 (2,9); 4,140 (8,7); 4,134 (8,2); 4,113 (3,2); 4,095 (1,2); 3,492 (6,3); 3,482 (5,6); 3,471 (6,1); 3,460 (11,6); 3,450 (8,6); 3,437 (7,6); 3,429 (8,2); 3,364 (1,2); 3,360 (0,9); 3,280 (10,6); 3,272 (11,4); 3,266 (10,4); 3,251 (5,2); 3,243 (7,8); 3,237 (8,3); 3,229 (7,5); 2,045 (13,8); 1,864 (0,6); 1,545 (157,1); 1,300 (1,2); 1,277 (5,5); 1,259 (16,0); 1,241 (5,7); 1,138 (0,7); 1,085 (0,7); 1,052 (0,6); 0,880 (3,5); 0,863 (1,9); 0,853 (2,1); 0,836 (1,5); 0,146 (0,8); 0,000 (282,3); -0,006 (4,3); 0,007 (3,6); -0,008 (8,6); 0,012 (1,7); -0,150 (0,9) |

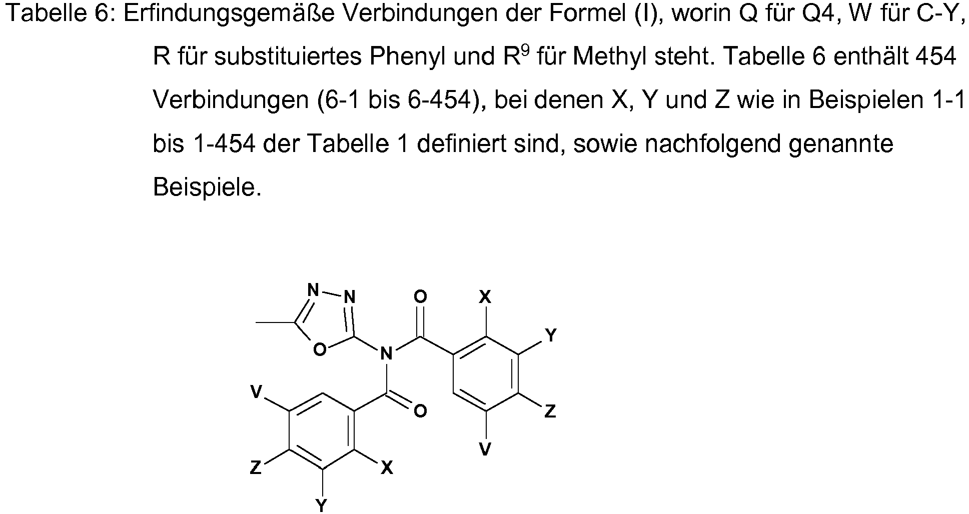

**Tabelle 7: Erfindungsgemäße Verbindungen der Formel (I) mit 2 identischen Acylresten, worin worin Q für Q1, W für N und R⁶ für Methyl steht.**

| | | | | |
|---|---|---|---|---|
| 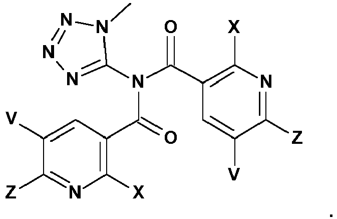 | | | | |

| Nr. | X | Z | V | Physikalische Daten (¹H-NMR, DMSO-d₆, 400 MHz) |
|---|---|---|---|---|
| 7-1 | F | CF₃ | H | |
| 7-2 | Cl | CF₃ | H | |
| 7-3 | Br | CF₃ | H | |
| 7-4 | Me | CF₃ | H | |
| 7-5 | CH₂OMe | CF₃ | H | |
| 7-6 | CH₂CH₂OCH₂OMe | CF₃ | H | |
| 7-7 | CH₂ -1,2-thiazolidin-1,1-dioxid | CF₃ | H | |
| 7-8 | CH₂-pyrrolidin-2-on | CF₃ | H | |
| 7-9 | F | F | H | |
| 7-10 | Cl | Cl | H | |
| 7-11 | Br | Br | H | |
| 7-12 | CF₃ | CF₃ | H | |
| 7-13 | F | SO₂Me | H | |
| 7-14 | Cl | SO₂Me | H | |
| 7-15 | Br | SO₂Me | H | |
| 7-16 | OMe | OMe | H | |

**Tabelle 8: Erfindungsgemäße Verbindungen der Formel (I), worin Q für Q1, W für N und R⁶ für Ethyl steht. Tabelle 8 enthält 16 Verbindungen (8-1 bis 8-16), bei denen X, Y und Z wie in Beispielen 7-1 bis 7-16 der Tabelle 7 definiert sind.**

| | | | | |
|---|---|---|---|---|
| 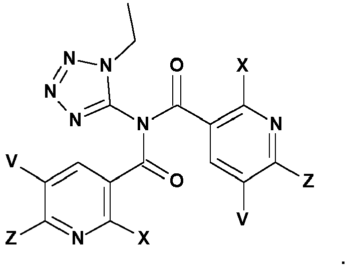 | | | | |

| Nr. | X | Z | V | Physikalische Daten (¹H-NMR, DMSO-d₆, 400 MHz) |
|---|---|---|---|---|
| 8-16 | OMe | OMe | H | 7.91 (d,2H), 6.45 (d,2H), 4.30 (q,2H), 3.86 (s,6H), 3.85 (s,6H), 1.45 (t,3H) |

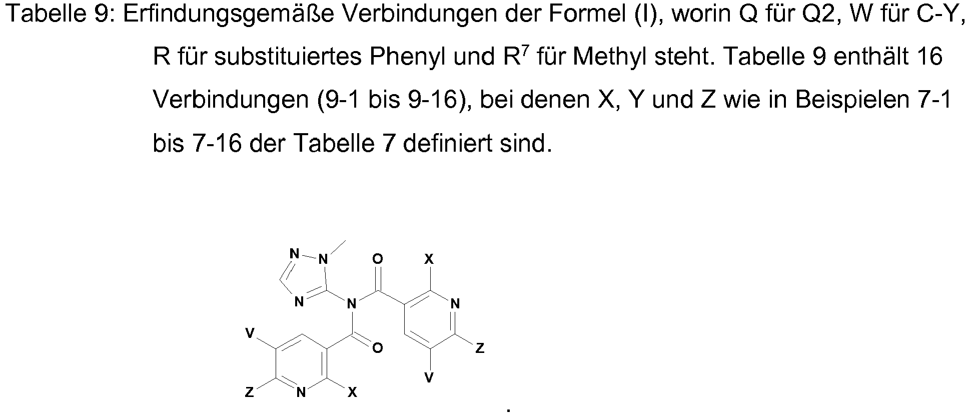

**Tabelle 10: Erfindungsgemäße Verbindungen der Formel (I), worin Q für Q3, W für N, R für substituiertes Phenyl und R⁸ für Methyl steht. Tabelle 10 enthält 16 Verbindungen (10-1 bis 10-16), bei denen X, Y und Z wie in Beispielen 7-1 bis 7-16 der Tabelle 7 definiert sind.**

| | | | | |
|---|---|---|---|---|
| 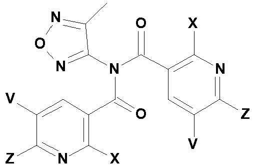 | | | | |

| Nr. | X | Z | V | Physikalische Daten (¹H-NMR, DMSO-d₆, 400 MHz) |
|---|---|---|---|---|
| 10-7 | CH₂-1,2-thiazolidin-1,1-dioxid | CF₃ | H | |

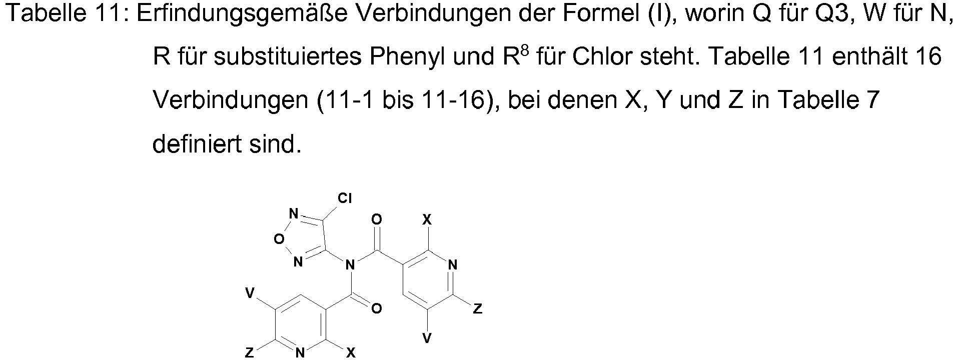

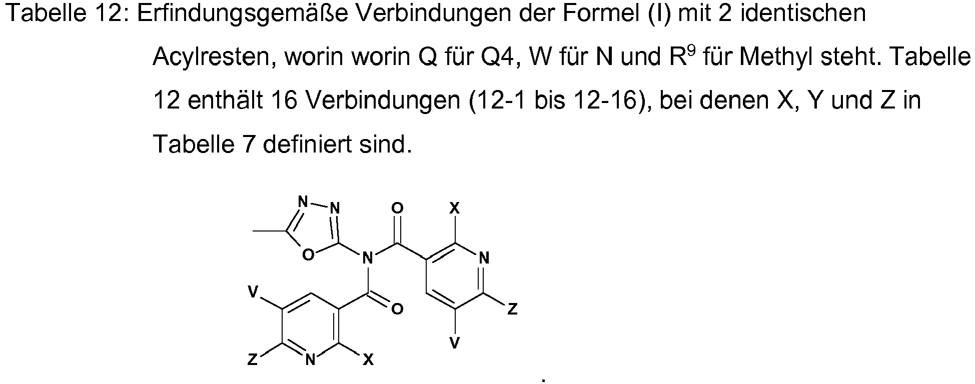

**Tabelle 13: Erfindungsgemäße Verbindungen der Formel (I), worin Q für Q1, Z für Methylsulfonyl, W für C-H , Z für Trifluormethyl, V für Wasserstoff und R⁶ für Methyl steht.**

| | | |
|---|---|---|
| 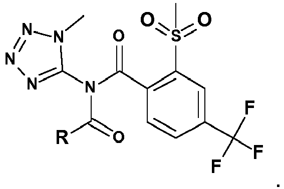 | | |

| Nr. | R | Physikalische Daten, (¹H-NMR, DMSO-d₆, 400 MHz) |
|---|---|---|
| 13-1 | Me | 8.36 (s,1H), 8.26 (d,1H), 8.06 (d,1H), 4.12 (s,3H), 3.38 (s,3H), 2.22 (s,3H) |
| 13-2 | Et | |
| 13-3 | i-Pr | |
| 13-4 | c-Pr | |
| 13-5 | CH₂CHMe₂ | |
| 13-6 | CF₃ | |
| 13-7 | CH₂CHF₂ | |
| 13-8 | CH₂CF₃ | |
| 13-9 | CH₂OMe | |
| 13-10 | CH₂SMe | |
| 13-11 | CH₂SO₂Me | |
| 13-12 | CH₂CH₂CN | |
| 13-13 | CH₂CH₂OMe | |
| 13-14 | CH₂CH₂OEt | |
| 13-15 | CH₂CH₂OPh | |
| 13-16 | CH₂CH₂SMe | |
| 13-17 | CH₂CH₂S(O)Me | |
| 13-18 | CH₂CH₂SO₂Me | |
| 13-19 | CH₂C(O)Me | |
| 13-20 | CH₂C(O)Ph | |
| 13-21 | CH₂CO₂H | |
| 13-22 | CH₂CO₂Me | |
| 13-23 | CH₂CN | |
| 13-24 | CH₂C(O)Me | |
| 13-25 | CH₂CO₂H | |
| 13-26 | CH₂CO₂Me | |
| 13-27 | CH₂CH₂CN | |
| 13-28 | Allyl | |
| 13-29 | Propargyl | |
| 13-30 | Phenyl | |
| 13-31 | 4-Cl-phenyl | 8.44 (d,1H), 8.40 (s,1H), 8.31 (d,1H), 7.63 (d,2H), 7.52 (d,2H), 4.22 (s,3H), 3.41 (s,3H) |
| 13-32 | 4-OMe-Ph | 8.40 (d,1H), 8.39 (s,1H), 8.29 (d,1H), 7.58 (d,2H), 6.97 (d,2H), 4.20 (s,3H), 3.79 (s,3H), 3.41 (s,3H) |
| 13-33 | Pyridin-3-yl | |
| 13-34 | Benzyl | |

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-086, 1-028, 1-206, 1-447, 1-448, 1-449, 1-454, 1-458, 1-470, 1-471, 1-472, 1-477, 1-487, 2-477, 2-480, 2-500, 3-143, 3-475, 3-491, 4-188, 4-495, 4-430, 4-496, 13-001, 13-031 und 13-032 bei einer Aufwandmenge von 320 g/ha jeweils eine mindestens 80 %-ige Wirkung gegen Abutilon theophrasti und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-028, 1-085, 1-086, 1-206, 1-447, 1-448, 1-449, 1-453, 1-457, 1-460, 1-470, 1-471, 1-472, 1-487, 2-500, 3-143, 3-146, 3-487, 3-491, 3-492, 4-144, 4-188, 13-001, 13-031 und 13-032 bei einer Aufwandmenge von 80 g/ha jeweils eine mindestens 80 %-ige Wirkung gegen Abutilon theophrasti und Veronica persica.

## Patentansprüche

1. Acylierte N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide oder deren Salze der Formel (I) 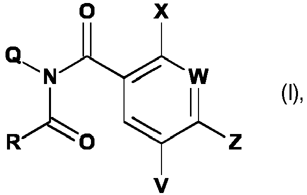 worin die Symbole und Indizes folgende Bedeutungen haben:
R bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Cycloalkyl-(C₁-C₆)-alkyl, (C₃-C₆)-Halogencycloalkyl-(C₁-C₆)-alkyl, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², COOR¹, CON(R¹)₂, oder jeweils durch s Reste aus der Gruppe bestehend aus X, Y, Z und V substituiertes Phenyl, Heteroaryl, Heterocyclyl oder Benzyl,
W bedeutet N oder CY,
X und Z bedeuten unabhängig voneinander jeweils Wasserstoff, Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹, OSO₂R² , S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C1-C6)-Alkyl-S(O)ₙR², (C1-C6)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, CH=NOR¹, (C₁-C₆)-Alkyl-CH=NOR¹, (C₁-C₆)-Alkyl-O-N=C(R¹)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die 6 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
oder
Y und Z bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils s Stickstoffatome, n Sauerstoffatome, n Schwefelatome und n Elemente S(O), S(O)₂, C=N-R¹⁷, C(OR¹⁷)₂, C[-O-(CH₂)₂-O-] oder C(O) als Ringglieder umfasst,
dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₁-C₆)-Alkoxy, Phenoxy, Halogen-(C₁-C₆)-alkoxy, (C₃-C₈)-Cycloalkyl, (C₂-C₈)-Alkoxyalkyl und Phenyl substituiert sind,
dessen Stickstoffatome durch n Reste aus der Gruppe bestehend aus (C₁-C₆)-Alkyl und Phenyl substituiert sind,
und worin die vorstehend genannten Phenylreste durch s Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
V bedeutet Wasserstoff, Nitro, Halogen, Cyano, (C₁-C₄)-Alkyl, (C₁-C₄)-Halogenalkyl, OR¹, S(O)ₙR²,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)NR⁴, N(R³)₂, NR³OR³, COR³, OCOR³, SCOR⁴, NR³COR³, NR³SO2R⁴ CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
R⁵ bedeutet (C₁-C₄)-Alkyl,
n bedeutet 0, 1 oder 2,
s bedeutet 0, 1, 2 oder 3,
Q bedeutet einen Rest Q1, Q2, Q3 oder Q4 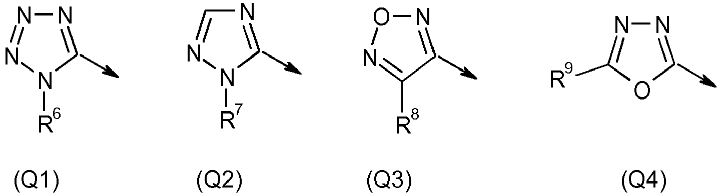
R⁶ und R⁷ bedeuten unabhängig voneinander (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei diese 6 vorstehend genannten Reste jeweils durch s Reste aus der Gruppe bestehend aus Nitro, Cyano, SiR¹²₃, PO(OR¹²)₃, S(O)ₙ-(C₁-C₆)-Alkyl, (C₁-C₆)-Alkoxy, Halogen-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-Cycloalkyl, Heteroaryl, Heterocyclyl, Phenyl, D-Heteroaryl, D-Heterocyclyl, D-Phenyl oder D-Benzyl substituiert sind, und wobei die 7 letztgenannten Reste durch s Reste aus der Gruppe Methyl, Ethyl, Methoxy, Trifluormethyl und Halogen substituiert sind, und wobei Heterocyclyl n Oxogruppen trägt,
oder
R⁶ und R⁷ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes (C₃-C₇)-Cycloalkyl, Heteroaryl, Heterocyclyl oder Phenyl,
R⁸ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfenyl, Methylsulfinyl, Methylsulfonyl, (C₁-C₆)-Alkylcarbonylamino, (C₁-C₆)-Alkoxycarbonylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁹ bedeutet Wasserstoff, (C₁-C₆)-Alkyl, R¹³O-(C₁-C₆)-Alkyl, CH₂R¹⁴, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹³, NHR¹³, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹¹ bedeutet (C₁-C₆)-Alkyl, (C₂-C₆)-Alkenyl, (C₂-C₆)-Alkinyl oder Phenyl,
R¹² bedeutet (C₁-C)-Alkyl,
R¹³ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁵-Heteroaryl oder (C₁-C₆)-Alkyl-NR¹⁵-Heterocyclyl, wobei die 21 letztgenannten Reste durch s Reste aus der Gruppe bestehend aus Cyano, Halogen, Nitro, Rhodano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ und (C₁-C₄)-Alkoxy-(C₂-C₆)-alkoxycarbonyl substituiert sind, und wobei Heterocyclyl n Oxogruppen trägt,
R¹⁴ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl;
R¹⁵ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁶ bedeutet (C₁-C₆)-Alkyl, (C₂-C₆)-Alkenyl oder (C₂-C₆)-Alkinyl,
R¹⁷ bedeutet (C₁-C₆)-Alkyl, Halogen-(C₁-C₆)-alkyl, (C₁-C₆)-Alkoxy und Halogen-(C₁-C₆)-alkoxy,
s bedeutet 0, 1, 2 oder 3,
n bedeutet 0, 1 oder 2,
D bedeutet O, S, oder NR¹¹,
mit der Maßgabe, daß V, X, Y und Z nicht gleichzeitig Wasserstoff bedeuten.

2. Acylierte N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide der Formel (I) nach Anspruch 1, worin
R bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Cycloalkyl-(C₁-C₆)-alkyl, (C₃-C₆)-Halogencycloalkyl-(C₁-C₆)-alkyl, (C₁-C₆)-AlkylS(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-C(O)R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², COOR¹, CON(R¹)₂, oder jeweils durch s Reste aus der Gruppe bestehend aus X, Y, Z und V substituiertes Phenyl, Heteroaryl, Heterocyclyl oder Benzyl,
W bedeutet N oder CY,
X und Z bedeuten unabhängig voneinander jeweils Wasserstoff, Halogen, (C₁-C₆)-Alkyl, (C₁-C₆)-Halogenalkyl, (C₂-C₆)-Alkenyl, (C₃-C₆)-Cycloalkyl, (C₃-C₆)-Halogencycloalkyl, OR¹, S(O)ₙR², SO₂N(R¹)₂, NR¹SO₂R², NR¹COR¹, (C₁-C₆)-AlkylS(O)ₙR², (C₁-C₆)-Alkyl-OR¹, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Wasserstoff, (C₂-C₆)-Alkenyl, COR¹, CO₂R¹, OCO₂R¹, NR¹CO₂R¹, C(O)N(R¹)₂, NR¹C(O)N(R¹)₂, OC(O)N(R¹)₂, C(O)N(R¹)OR¹, NR¹SO₂R², NR¹COR¹, OR¹, S(O)ₙR², SO₂N(R¹)₂, (C₁-C₆)-Alkyl-S(O)ₙR², (C₁-C₆)-Alkyl-OR¹, (C₁-C₆)-Alkyl-OCOR¹, (C₁-C₆)-Alkyl-CO₂R¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, CH=NOR¹, (C₁-C₆)-Alkyl-CH=NOR¹, (C₁-C₆)-Alkyl-Heteroaryl, (C₁-C₆)-Alkyl-Heterocyclyl, Heteroaryl oder Heterocyclyl, wobei die 4 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
V bedeutet Wasserstoff, Cl, OMe, Methyl oder Ethyl,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R^{4,} CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl,
n bedeutet 0, 1 oder 2,
s bedeutet 0, 1, 2 oder 3,
Q bedeutet einen Rest Q1, Q2, Q3 oder Q4 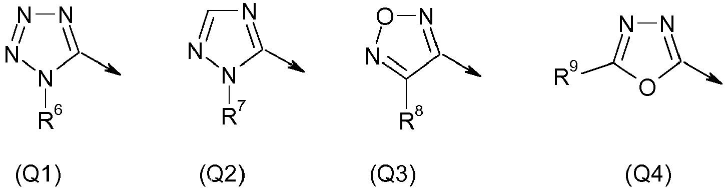 R⁶ und R⁷ bedeuten unabhängig voneinander (C₁-C₆)-Alkyl, Halogen-(C₁-C₆)-alkyl, (C₂-C₆)-Alkenyl, wobei diese 3 vorstehend genannten Reste jeweils durch s (C₁-C₆)-Alkoxy-Reste substituiert sind,
R⁸ bedeutet Chlor, Methyl, Methoxymethyl, Amino oderAcetylamino,
R⁹ bedeutet Methyl, Ethyl, Methoxymethyl oder Methoxyethyl.

3. Acylierte N-(1,2,5-Oxadiazol-3-yl)-, N-(1,3,4-Oxadiazol-2-yl)-, N-(Tetrazol-5-yl)- und N-(Triazol-5-yl)-arylcarbonsäureamide der Formel (I) nach Anspruch 1 oder 2, worin
R bedeutet Methyl oder jeweils durch s Reste aus der Gruppe bestehend aus X, Y und Z substituiertes Phenyl,
W bedeutet CY,
X bedeutet F, Cl, Br, Methyl, Ethyl, Cyclopropyl, Trifluormethyl, Methoxy, Methoxymethyl, Methoxyethoxymethyl, SMe oder SO₂Me,
Z bedeutet Wasserstoff, F, Cl, Br, I, Methyl, Ethyl, Trifluormethyl, Difluormethyl, Pentafluorethyl, Methylsulfonyl oder Ethylsulfonyl,
Y bedeutet Wasserstoff, SMe, S(O)Me, SO₂Me, SEt, S(O)Et, SO₂Et, CH₂OMe, CH₂OEt, CH₂OCH₂CF₃, ,CH₂SMe, CH₂S(O)Me, CH₂SO₂Me, Vinyl, C(O)Me, C(O)Et, C(O)cPr, CO₂Me, CHN=OMe, 4,5-dihydro-1,2-oxazol-3-yl, 5-methyl-4,5-dihydro-1,2-oxazol-3-yl, 5-methyl-4,5-dihydro-1,2-oxazol-3-yl, 5-cyanomethyl-4,5-dihydro-1,2-oxazol-3-yl, 4,5-dihydro-1,2-oxazol-5-yl, 3-methyl-4,5-dihydro-1,2-oxazol-5-yl, 1H-pyrazol-1-yl, 1H-1,2,3-triazol-1-yl, 2H-1,2,3-triazol-2-yl, 1H-1,2,4-triazol-1-yl, pyrolidin-2-on-1-yl, morpholin-3-on-4-yl, OMe, OEt, OnPr, OCH₂cPr, OCH₂CH₂F, OCH₂CH₂OMe oder OCH₂CH₂CH₂OMe,
V bedeutet Wasserstoff,
Q bedeutet einen Rest Q1, Q2, Q3 oder Q4 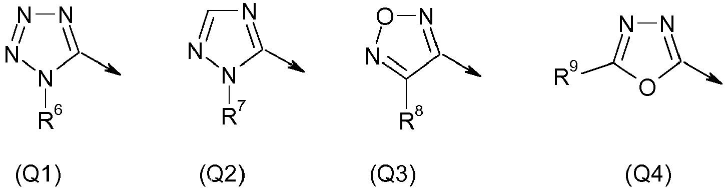
R⁶ bedeutet Methyl oder Ethyl,
R⁷ bedeutet Methyl,
R⁸ bedeutet Chlor oder Methyl,
R⁹ bedeutet Methyl,
s bedeutet 0,1,2 oder 3.

4. Herbizide Mittel, **gekennzeichnet durch** einen herbizid wirksamen Gehalt an mindestens einem acylierten N-(1,2,5-Oxadiazol-3-yl)-, N-(1,3,4-Oxadiazol-2-yl)-, N-(Tetrazol-5-yl)- oder N-(Triazol-5-yl)-arylcarbonsäureamide gemäß einem der Ansprüche 1 bis 3.

5. Herbizide Mittel nach Anspruch 4 in Mischung mit Formulierungshilfsmitteln.

6. Herbizide Mittel nach Anspruch 5 enthaltend ein weiteres Herbizid.

7. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 6 auf die Pflanzen oder auf den Ort des unerwünschten Pflanzenwachstums appliziert.

8. Verwendung von Verbindungen der Formel (I) gemäß einem der Ansprüche 1 bis 3 oder von herbiziden Mitteln nach einem der Ansprüche 4 bis 6 zur Bekämpfung unerwünschter Pflanzen.

9. Verwendung nach Anspruch 8, **dadurch gekennzeichnet, daß** die Verbindungen der Formel (I) zur Bekämpfung unerwünschter Pflanzen in Kulturen von Nutzpflanzen eingesetzt werden.

10. Verwendung nach Anspruch 9, **dadurch gekennzeichnet, daß** die Nutzpflanzen transgene Nutzpflanzen sind.

## Claims

1. Acylated N-(1,2,5-oxadiazol-3-yl)-, N-(1,3,4-oxadiazol-2-yl)-, N-(tetrazol-5-yl)- and N-(triazol-5-yl)arylcarboxamides, or salts thereof, of the formula (I) 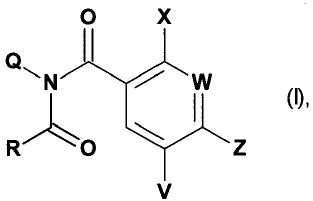 in which the symbols and indices are defined as follows:
R is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-cycloalkyl-(C₁-C₆)-alkyl, (C₃-C₆)-halocycloalkyl- (C₁-C₆)-alkyl, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-COOR¹, (C₁-C₆)-alkyl-C(O)R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², COOR¹, CON(R¹)₂, or phenyl, heteroaryl, heterocyclyl or benzyl, each substituted by s radicals from the group consisting of X, Y, Z and V,
W is N or CY,
X and Z are each independently hydrogen, nitro, halogen, cyano, formyl, thiocyanato, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-halocycloalkyl, (C₃-C₆)-cycloalkyl- (C₁-C₆)-alkyl, (C₃-C₆)-halocycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-COOR¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR₁R₂, P(O)(OR⁵)₂, or
heteroaryl, heterocyclyl or phenyl, each substituted by s radicals from the group of methyl, ethyl, methoxy, nitro, trifluoromethyl and halogen,
Y is hydrogen, nitro, halogen, cyano, thiocyanato, (C₁-C₆)-alkyl, halo- (C₁-C₆)-alkyl, (C₂-C₆)-alkenyl, halo-(C₂-C₆)-alkenyl, (C₂-C₆)-alkynyl, halo- (C₂-C₆)-alkynyl, (C₃-C₆)-cycloalkyl, (C₃-C₆)-cycloalkenyl, halo- (C₃-C₆)-cycloalkyl, (C₃-C₆)-cycloalkyl- (C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂ (C₁-C₆)-alkyl-S(O)ₙR², (C₁-C₆)-alkyl-OR¹, (C₁-C₆)-alkyl-OCOR¹, (C₁-C₆)-alkyl-OSO₂R². (C₁-C₆)-alkyl-CO₂R¹, (C₁-C₆)-alkyl-CN, (C₁-C₆)-alkyl-SO₂OR¹, (C₁-C₆)-alkyl-CON (R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, CH=NOR¹, (C₁-C₆)-alkyl-CH=NOR¹, (C₁-C₆)-alkyl-O-N=C(R¹)₂, (C₁-C₆)-alkylphenyl, (C₁-C₆)-alkylheteroaryl, (C₁-C₆)-alkylheterocyclyl, phenyl, heteroaryl or heterocyclyl, where the 6 latter radicals are each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy, halo- (C₁-C₆)-alkoxy, (C₁-C₆)-alkoxy-(C₁-C₄)-alkyl and cyanomethyl, and where heterocyclyl bears n oxo groups,
or
Y and Z together with the two atoms to which they are bonded form a 5-, 6- or 7-membered, unsaturated, partly saturated or saturated ring which, as well as carbon atoms, in each case comprises s nitrogen atoms, n oxygen atoms, n sulfur atoms and n S(O), S(O)₂, C=N-R¹⁷, C(OR¹⁷)₂, C[-O-(CH₂)₂-O-] or C(O) elements as ring members,
wherein the carbon atoms are substituted by s radicals from the group consisting of halogen, cyano, (C₁-C₆)-alkyl, (C₂-C₁₀)-alkenyl, (C₂-C₁₀)-alkynyl, (C₁-C₆)-haloalkyl, (C₁-C₆)-alkoxy, phenoxy, halo- (C₁-C₆)-alkoxy, (C₃-C₈) -cycloalkyl, (C₂-C₈)-alkoxyalkyl and phenyl,
wherein the nitrogen atoms are substituted by n radicals from the group consisting of (C₁-C₆)-alkyl and phenyl,
and in which the aforementioned phenyl radicals are substituted by s radicals from the group consisting of cyano, nitro, halogen, (C₁-C₆)-alkyl, (C₁-C₆)-haloalkyl and (C₁-C₆)-alkoxy,
V is hydrogen, nitro, halogen, cyano, (C₁-C₄)-alkyl, (C₁-C₄)-haloalkyl, OR¹, S(O)ₙR²,
R¹ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-0-(C₁-C₆)-alkyl, (C₃-C₆)-cycloalkyl- (C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl bears n oxo groups,
R² is (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl- (C₁-C₆)-alkyl, phenyl, phenyl- (C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl bears n oxo groups,
R³ is hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl,
R⁴ is (C₁-C₆)-alkyl, (C₂-C₆)-alkenyl or (C₂-C₆)-alkynyl, (C₃-C₆)-cycloalkyl or (C₃-C₆)-cycloalkyl- (C₁-C₆)-alkyl,
R⁵ is (C₁-C₄)-alkyl,
n is 0, 1 or 2,
s is 0, 1, 2 or 3,
Q is a Q1, Q2, Q3 or Q4 radical 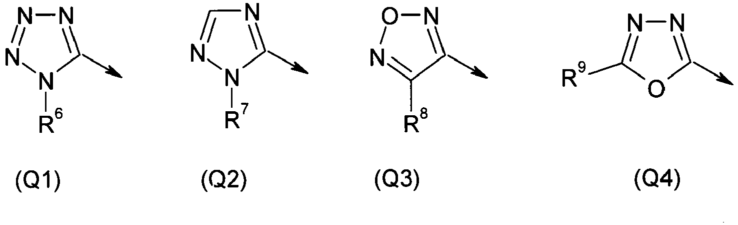
R⁶ and R⁷ are independently (C₁-C₆)-alkyl, halo- (C₁-C₆)-alkyl, (C₂-C₆)-alkenyl, halo- (C₂-C₆)-alkenyl, (C₂-C₆)-alkynyl, halo- (C₂-C₆)-alkynyl,
where these 6 aforementioned radicals are each substituted by s radicals from the group consisting of nitro, cyano, SiR¹²₃, PO(OR¹²)₃, S(O)ₙ-(C₁-C₆)-alkyl, (C₁-C₆)-alkoxy, halo-(C₁-C₆)-alkoxy, N(R¹⁰)₂, COR¹⁰, COOR¹⁰, OCOR¹⁰, OCO₂R¹⁰, NR¹⁰COR¹⁰, NR¹⁰SO₂R¹¹, (C₃-C₆)-cycloalkyl, heteroaryl, heterocyclyl, phenyl, D-heteroaryl, D-heterocyclyl, D-phenyl or D-benzyl, and where the 7 latter radicals are substituted by s radicals from the group of methyl, ethyl, methoxy, trifluoromethyl and halogen, and where heterocyclyl bears n oxo groups,
or
R⁶ and R⁷ are each (C₃-C₇)-cycloalkyl, heteroaryl, heterocyclyl or phenyl, each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy, halo- (C₁-C₆)-alkoxy and (C₁-C₆)-alkoxy-(C₁-C₄)-alkyl,
R⁸ is hydrogen, (C₁-C₆)-alkyl, (C₃-C₇)-cycloalkyl, halo- (C₁-C₆)-alkyl, (C₁-C₆)-alkoxy, halo- (C₁-C₆)-alkoxy, (C₂-C₆)-alkenyl, (C₂-C₆)-alkenyloxy, halo- (C₂-C₆)-alkenyl, (C₂-C₆)-alkynyl, (C₂-C₆)-alkynyloxy, halo- (C₂-C₆)-alkynyl, cyano, nitro, methylsulfenyl, methylsulfinyl, methylsulfonyl, (C₁-C₆)-alkylcarbonylamino, (C₁-C₆)-alkoxycarbonylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or heteroaryl, heterocyclyl or phenyl, each substituted by s radicals from the group consisting of methyl, ethyl, methoxy, trifluoromethyl and halogen;
R⁹ is hydrogen, (C₁-C₆)-alkyl, R¹³O-(C₁-C₆)-alkyl, CH₂R¹⁴, (C₃-C₇)-cycloalkyl, halo- (C₁-C₆)-alkyl, (C₂-C₆)-alkenyl, halo- (C₂-C₆)-alkenyl, (C₂-C₆)-alkynyl, halo-(C₂-C₆)-alkynyl, OR¹³, NHR¹³, methoxycarbonyl, ethoxycarbonyl, methoxycarbonylmethyl, ethoxycarbonylmethyl, methylcarbonyl, trifluoromethylcarbonyl, dimethylamino, acetylamino, methylsulfenyl, methylsulfinyl, methylsulfonyl, or heteroaryl, heterocyclyl, benzyl or phenyl, each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy, halo- (C₁-C₆)-alkoxy and (C₁-C₆)-alkoxy-(C₁-C₄)-alkyl,
R¹⁰ is hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl or phenyl,
R¹¹ is (C₁-C₆)-alkyl, (C₂-C₆)-alkenyl, (C₂-C₆)-alkynyl or phenyl,
R¹² is (C₁-C)-alkyl,
R¹³ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl- (C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR¹⁵-heteroaryl or (C₁-C₆)-alkyl-NR¹⁵-heterocyclyl, where the 21 latter radicals are substituted by s radicals from the group consisting of cyano, halogen, nitro, thiocyanato, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ and (C₁-C₄)-alkoxy- (C2-C₆)-alkoxycarbonyl, and where heterocyclyl bears n oxo groups,
R¹⁴ is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₃-C₆)-alkoxy, (C₃-C₆)-cycloalkyl, or heteroaryl, heterocyclyl or phenyl, each substituted by s radicals from the group consisting of methyl, ethyl, methoxy, trifluoromethyl and halogen;
R¹⁵ is hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl,
R¹⁶ is (C₁-C₆)-alkyl, (C₂-C₆)-alkenyl or (C₂-C₆)-alkynyl,
R¹⁷ is (C₁-C₆)-alkyl, halo- (C₁-C₆)-alkyl, (C₁-C₆)-alkoxy and halo-(C₁-C₆)-alkoxy,
s is 0, 1, 2 or 3,
n is 0, 1 or 2,
D is 0, S, or NR¹¹,
with the proviso that V, X, Y and Z are not simultaneously hydrogen.

2. Acylated N-(1,2,5-oxadiazol-3-yl)-, N-(1,3,4-oxadiazol-2-yl)-, N-(tetrazol-5-yl)- and N-(triazol-5-yl)arylcarboxamides of the formula (I) according to Claim 1, in which
R is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-cycloalkyl-(C₁-C₆)-alkyl, (C₃-C₆)-halocycloalkyl- (C₁-C₆)-alkyl, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-COOR¹, (C₁-C₆)-alkyl-C(O)R¹, (C₁-C₆)-alkyl-CN, (C₁-C₆)-alkyl-SO₂OR¹, (C₁-C₆)-alkyl-CON (R¹)₂, (C₁-C₆)-alkyl-SO₂N(R¹)₂, (C₁-C₆)-alkyl-NR¹COR¹, (C₁-C₆)-alkyl-NR¹SO₂R², COOR¹, CON(R¹)₂, or phenyl, heteroaryl, heterocyclyl or benzyl, each substituted by s radicals from the group consisting of X, Y, Z and V,
W is N or CY,
X and Z are each independently hydrogen, halogen, (C₁-C₆)-alkyl, (C₁-C₆)-haloalkyl, (C₂-C₆)-alkenyl, (C₃-C₆)-cycloalkyl, (C₃-C₆)-halocycloalkyl, OR¹, S(O)ₙR², SO₂N(R¹)₂, NR¹SO₂R², NR¹COR¹, (C₁-C₆)-alkyl-S(O)ₙR², (C₁-C₆)-alkyl-OR¹, or
heteroaryl, heterocyclyl or phenyl, each substituted by s radicals from the group of methyl, ethyl, methoxy, nitro, trifluoromethyl and halogen,
Y is hydrogen, (C₂-C₆)-alkenyl, COR¹, CO₂R¹, OCO₂R¹, NR¹CO₂R¹, C(O)N(R¹)₂, NR¹C(O)N(R¹)₂, OC(O)N(R¹)₂, C(O)N(R¹)OR¹, NR¹SO₂R², NR¹COR¹, OR¹, S(O)ₙR², SO₂N(R¹)₂, (C₁-C₆)-alkyl-S(O)ₙR², (C₁-C₆)-alkyl-OR¹, (C₁-C₆)-alkyl-OCOR¹, (C₁-C₆)-alkyl-CO₂R¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, CH=NOR¹, (C₁-C₆)-alkyl-CH=NOR¹, (C₁-C₆)-alkylheteroaryl, (C₁-C₆)-alkylheterocyclyl, heteroaryl or heterocyclyl, where the 4 latter radicals are each substituted by s radicals from the group consisting of halogen, nitro, cyano, (C₁-C₆) -alkyl, halo- (C₁-C₆) -alkyl, (C₃-C₆)-cycloalkyl, S(O)ₙ-(C₁₋C₆)-alkyl, (C₁-C₆) -alkoxy, halo- (C₁-C₆)-alkoxy, (C₁-C₆) -alkoxy- (C₁-C₄) -alkyl and cyanomethyl, and where heterocyclyl bears n oxo groups,
V is hydrogen, Cl, OMe, methyl or ethyl,
R¹ is hydrogen, (C₁-C₆)-alkyl, (C₁-C₆)-haloalkyl, (C₂-C₆) -alkenyl, (C₂-C₆) -haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆) -alkyl-0-(C₁-C₆)-alkyl, (C₃-C₆) -cycloalkyl- (C₁-C₆) -alkyl, phenyl, phenyl-(C₁-C₆)-alkyl, heteroaryl, (C₁-C₆) - alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy- (C₂-C₆)-alkoxycarbonyl, and where heterocyclyl bears n oxo groups,
R² is (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆) -cycloalkenyl, (C₃-C₆)-halocycloalkyl, (C₁-C₆)-alkyl-O-(C₁-C₆) -alkyl, (C₃-C₆)-cycloalkyl-(C₁-C₆)-alkyl, phenyl, phenyl-(C₁-C₆) - alkyl, heteroaryl, (C₁-C₆)-alkylheteroaryl, heterocyclyl, (C₁-C₆) -alkylheterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 latter radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl bears n oxo groups,
R³ is hydrogen, (C₁-C₆)-alkyl, (C₂-C₆)-alkenyl, (C₂-C₆)-alkynyl, (C₃-C₆) -cycloalkyl or (C₃-C₆) -cycloalkyl-(C₁-C₆)-alkyl,
R⁴ is (C₁-C₆) -alkyl, (C₂-C₆) -alkenyl or (C₂-C₆)-alkynyl, (C₃-C₆)-cycloalkyl or (C₃-C₆)-cycloalkyl-(C₁-C₆)-alkyl,
n is 0, 1 or 2,
s is 0, 1, 2 or 3,
Q is a Q1, Q2, Q3 or Q4 radical 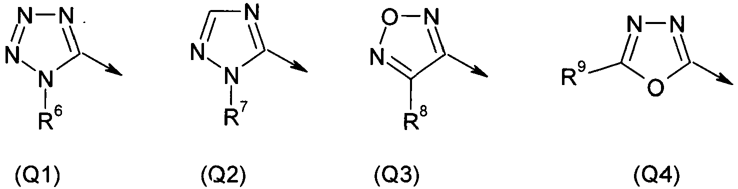
R⁶ and R⁷ are independently (C₁-C₆)-alkyl, halo-(C₁-C₆)-alkyl, (C₂-C₆)-alkenyl, where these 3 aforementioned radicals are each substituted by s (C₁-C₆)-alkoxy radicals,
R⁸ is chlorine, methyl, methoxymethyl, amino or acetylamino,
R⁹ is methyl, ethyl, methoxymethyl or methoxyethyl.

3. Acylated N-(1,2,5-oxadiazol-3-yl)-, N-(1,3,4-oxadiazol-2-yl)-, N-(tetrazol-5-yl)- and N-(triazol-5-yl)arylcarboxamides of the formula (I) according to Claim 1 or 2, in which
R is methyl, or phenyl substituted in each case by s radicals from the group consisting of X, Y and Z,
W is CY,
X is F, Cl, Br, methyl, ethyl, cyclopropyl, trifluoromethyl, methoxy, methoxymethyl, methoxyethoxymethyl, SMe or SO₂Me,
Z is hydrogen, F, Cl, Br, I, methyl, ethyl, trifluoromethyl, difluoromethyl, pentafluoroethyl, methylsulfonyl or ethylsulfonyl,
Y is hydrogen, SMe, S(O)Me, SO₂Me, SEt, S(O)Et, SO₂Et, CH₂OMe, CH₂OEt, CH₂OCH₂CF₃, CH₂SMe, CH₂S(O)Me, CH₂SO₂Me, vinyl, C(O)Me, C(O)Et, C(O)cPr, CO₂Me, CHN=OMe, 4,5-dihydro-1,2-oxazol-3-yl, 5-methyl-4,5-dihydro-1,2-oxazol-3-yl, 5-methyl-4,5-dihydro-1,2-oxazol-3-yl, 5-cyanomethyl-4,5-dihydro-1,2-oxazol-3-yl, 4,5-dihydro-1,2-oxazol-5-yl, 3-methyl-4,5-dihydro-1,2-oxazol-5-yl, 1H-pyrazol-1-yl, 1H-1,2,3-triazol-1-yl, 2H-1,2,3-triazol-2-yl, 1H-1,2,4-triazol-1-yl, pyrolidin-2-on-1-yl, morpholin-3-on-4-yl, OMe, OEt, OnPr, OCH₂cPr, OCH₂CH₂F, OCH₂CH₂OMe or OCH₂CH₂CH₂OMe,
V is hydrogen,
Q is a Q1, Q2, Q3 or Q4 radical 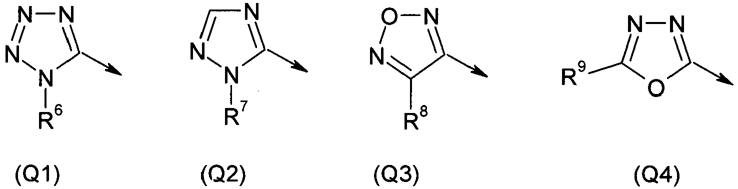
R⁶ is methyl or ethyl,
R⁷ is methyl,
R⁸ is chlorine or methyl,
R⁹ is methyl,
s is 0, 1, 2 or 3.

4. Herbicidal compositions, **characterized by** a herbicidally active content of at least one acylated N-(1,2,5-oxadiazol-3-yl)-, N-(1,3,4-oxadiazol-2-yl)-, N-(tetrazol-5-yl)- or N-(triazol-5-yl)-aryl carboxamides according to any of Claims 1 to 3.

5. Herbicidal compositions according to Claim 4 in a mixture with formulation auxiliaries.

6. Herbicidal compositions according to Claim 5 comprising a further herbicide.

7. Method of controlling unwanted plants, **characterized in that** an effective amount of at least one compound of the formula (I) according to any of Claims 1 to 3 or of a herbicidal composition according to any of Claims 4 to 6 is applied to the plants or to the site of the unwanted vegetation.

8. Use of compounds of the formula (I) according to any of Claims 1 to 3 or of herbicidal compositions according to any of Claims 4 to 6 for controlling unwanted plants.

9. Use according to Claim 8, **characterized in that** the compounds of the formula (I) are used for controlling unwanted plants in crops of useful plants.

10. Use according to Claim 9, **characterized in that** the useful plants are transgenic useful plants.

## Revendications

1. Amides d'acides N-(1,2,5-oxadiazol-3-yl)-, N-(1,3,4-oxadiazol-2-yl)-, N-(tétrazol-5-yl)- et N-(triazol-5-yl)-arylcarboxyliques acylés ou leurs sels de la formule (I) : 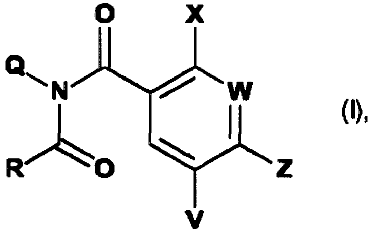 dans laquelle les symboles et les indices ont les significations suivantes :
R signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₃-C₆), cycloalkyle en (C₃-C₆), cycloalkyle en (C₃-C₆)-alkyle en (C₁-C₆), halogénocycloalkyle en (C₃-C₆)-alkyle en (C₁-C₆), alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-COOR¹, alkyle en (C₁-C₆)-C(O)R¹, alkyle en (C₁-C₆)-CN, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², COOR¹, CON(R¹)₂, ou phényle, hétéroaryle, hétérocyclyle ou benzyle, chacun substitués par s radicaux du groupe constitué par X, Y,
Z et V,
W signifie N ou CY,
X et Z signifient chacun indépendamment l'un de l'autre hydrogène, nitro, halogène, cyano, formyle, rhodano, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₃-C₆), cycloalkyle en (C₃-C₆), halogénocycloalkyle en (C₃-C₆), cycloalkyle en (C₃-C₆)-alkyle en (C₁-C₆), halogénocycloalkyle en (C₃-C₆)-alkyle en (C₁-C₆), COR¹, OR¹, OCOR¹, OSO₂R², S(O)ₙR², SO₂OR¹, SO₂N(R¹)₂, NR¹SO₂R², NR¹COR¹, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-COOR¹, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², NR₁R₂, P(O)(OR⁵)₂,
ou
hétéroaryle, hétérocyclyle ou phényle, chacun substitués par s radicaux du groupe constitué par méthyle, éthyle, méthoxy, nitro, trifluorométhyle et halogène,
Y signifie hydrogène, nitro, halogène, cyano, rhodano, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, OCOOR¹, NR¹COOR¹, C(O)N(R¹)₂, NR¹C (O)N(R¹)₂, OC(O)N(R¹)₂, C(O)N(R¹)OR¹, NR¹SO₂R², NR¹COR¹, OR¹, OSO₂R², S(O)ₙR², SO₂OR¹, SO₂N(R¹)₂, alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-CN, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², N(R¹)₂, P(O)(OR⁵)₂, CH₂P(O)(OR⁵)₂, CH=NOR¹, alkyle en (C₁-C₆)-CH=NOR¹, alkyle en (C₁-C₆)-O-N=C(R¹)₂, alkyle en (C₁-C₆)-phényle, alkyle en (C₁-C₆)-hétéroaryle, alkyle en (C₁-C₆)-hétérocyclyle, phényle, hétéroaryle ou hétérocyclyle, les 6 derniers radicaux cités étant chacun substitués par s radicaux du groupe constitué par halogène, nitro, cyano, alkyle en (C₁-C₆), halogénoalkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄) et cyanométhyle, et l'hétérocyclyle portant n groupes oxo,
ou
Y et Z forment ensemble avec les deux atomes auxquels ils sont reliés un cycle à 5, 6 ou 7 chaînons, insaturé, partiellement saturé ou saturé, qui comprend en plus des atomes de carbone respectivement s atomes d'azote, n atomes d'oxygène, n atomes de soufre et n éléments S(O), S(O)₂, C=N-R¹⁷, C(OR¹⁷)₂, C[-O-(CH₂)₂-O-] ou C(O) en tant que chaînons,
dont les atomes de carbone sont substitués par s radicaux du groupe constitué par halogène, cyano, alkyle en (C₁-C₆), alcényle en (C₂-C₁₀), alcynyle en (C₂-C₁₀), haloalkyle en (C₁-C₆), alcoxy en (C₁-C₆), phénoxy, halogéno-alcoxy en (C₁-C₆), cycloalkyle en (C₃-C₈), alcoxyalkyle en (C₂-C₈) et phényle,
dont les atomes d'azote sont substitués par n radicaux du groupe constitué par alkyle en (C₁-C₆) et phényle,
et où les radicaux phényle mentionnés précédemment sont substitués par s radicaux du groupe constitué par cyano, nitro, halogène, alkyle en (C₁-C₆), haloalkyle en (C₁-C₈) et alcoxy en (C₁-C₆),
V signifie hydrogène, nitro, halogène, cyano, alkyle en (C₁-C₄), halogénoalkyle en (C₁-C₄), OR¹, S(O)ₙR²,
R¹ signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et alcoxy en (C₁-C₄)-alcoxycarbonyle en (C₂-C₆), et l'hétérocyclyle portant n groupes oxo,
R² signifie alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et alcoxy en (C₁-C₄)-alcoxycarbonyle en (C₂-C₆), et l'hétérocyclyle portant n groupes oxo,
R³ signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆),
R⁴ signifie alkyle en (C₁-C₆), alcényle en (C₂-C₆) ou alcynyle en (C₂-C₆), cycloalkyle en (C₃-C₆) ou cycloalkyle en (C₃-C₆)-alkyle en (C₁-C₆),
R⁵ signifie alkyle en (C₁-C₄),
n signifie 0, 1 ou 2,
s signifie 0, 1, 2 ou 3,
Q signifie un radical Q1, Q2, Q3 ou Q4 : 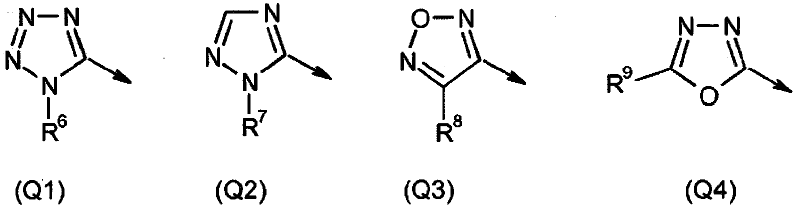
R⁶ et R⁷ signifient indépendamment l'un de l'autre alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), ces 6 radicaux mentionnés précédemment étant chacun substitués par s radicaux du groupe constitué par nitro, cyano, SiR¹²₃, PO(OR¹²)₃, S(O)ₙ-alkyle en (C₁-C₆), alcoxy en (C₁-C₆), halogéno-alcoxy en (C₁-C₆), N(R¹⁰)₂, COR¹⁰, COOR¹⁰, OCOR¹⁰, OCO₂R¹⁰, NR¹⁰COR¹⁰, NR¹⁰SO₂R¹¹, cycloalkyle en (C₃-C₆), hétéroaryle, hétérocyclyle, phényle, D-hétéroaryle, D-hétérocyclyle, D-phényle ou D-benzyle, et les 7 derniers radicaux cités étant substitués par s radicaux du groupe constitué par méthyle, éthyle, méthoxy, trifluorométhyle et halogène, et l'hétérocyclyle portant n groupes oxo,
ou
R⁶ et R⁷ signifient chacun cycloalkyle en (C₃-C₇), hétéroaryle, hétérocyclyle ou phényle, chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆) et alcoxy en (C₁-C₆)-alkyle en (C₁-C₄),
R⁸ signifie hydrogène, alkyle en (C₁-C₆), cycloalkyle en (C₃-C₇), halogéno-alkyle en (C₁-C₆), alcoxy en (C₁-C₆), halogéno-alcoxy en (C₁-C₆), alcényle en (C₂-C₆), alcényloxy en (C₂-C₆), halogéno-alcényle en (C₂-C₆), alcynyle en (C₂-C₆), alcynyloxy en (C₂-C₆), halogéno-alcynyle en (C₂-C₆), cyano, nitro, méthylsulfényle, méthylsulfinyle, méthylsulfonyle, alkylcarbonylamino en (C₁-C₆), alcoxycarbonylamino en (C₁-C₆), benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogène, amino, aminocarbonyle, méthylaminocarbonyle, diméthylaminocarbonyle, méthoxyméthyle, ou hétéroaryle, hétérocyclyle ou phényle, chacun substitués par s radicaux du groupe constitué par méthyle, éthyle, méthoxy, trifluorométhyle et halogène ;
R⁹ signifie hydrogène, alkyle en (C₁-C₆), R¹³O-alkyle en (C₁-C₆), CH₂R¹⁴, cycloalkyle en (C₃-C₇), halogéno-alkyle en (C₁-C₆), alcényle en (C₂-C₆), halogéno-alcényle en (C₂-C₆), alcynyle en (C₂-C₆), halogéno-alcynyle en (C₂-C₆), OR¹³, NHR¹³, méthoxycarbonyle, éthoxycarbonyle, méthoxycarbonylméthyle, éthoxycarbonylméthyle, méthylcarbonyle, trifluorométhylcarbonyle, diméthylamino, acétylamino, méthylsulfényle, méthylsulfinyle, méthylsulfonyle, ou hétéroaryle, hétérocyclyle, benzyle ou phényle, chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆) et alcoxy en (C₁-C₆)-alkyle en (C₁-C₄),
R¹⁰ signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆) ou phényle,
R¹¹ signifie alkyle en (C₁-C₆), alcényle en (C₂-C₆), alcynyle en (C₂-C₆) ou phényle,
R¹² signifie alkyle en (C₁-C),
R¹³ signifie hydrogène, alkyle en (C₁-C₆), halogénoalkyle en (C₁-C₆), alcényle en (C₂-C₆), halogéno-alcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR¹⁵-hétéroaryle ou alkyle en (C₁-C₆)-NR¹⁵-hétérocyclyle, les 21 derniers radicaux cités étant substitués par s radicaux du groupe constitué par cyano, halogène, nitro, rhodano, OR¹⁵, S(O)ₙR¹⁶, N(R¹⁵)₂, NR¹⁵OR¹⁵, COR¹⁵, OCOR¹⁵, SCOR¹⁶, NR¹⁵COR¹⁵, NR¹⁵SO₂R¹⁶, CO₂R¹⁵, COSR¹⁶, CON(R¹⁵)₂ et alcoxy en (C₁-C₄)-alcoxycarbonyle en (C₂-C₆), et l'hétérocyclyle portant n groupes oxo,
R¹⁴ signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, alcoxy en (C₃-C₆), cycloalkyle en (C₃-C₆), ou hétéroaryle, hétérocyclyle ou phényle, chacun substitués par s radicaux du groupe constitué par méthyle, éthyle, méthoxy, trifluorométhyle et halogène ;
R¹⁵ signifie hydrogène, alkyle en (C₁-C₆), alcényle en (C₂-C₆), alcynyle en (C₃-C₆), cycloalkyle en (C₃-C₆) ou cycloalkyle en (C₃-C₆)-alkyle en (C₁-C₆),
R¹⁶ signifie alkyle en (C₁-C₆), alcényle en (C₂-C₆) ou alcynyle en (C₂-C₆),
R¹⁷ signifie alkyle en (C₁-C₆), halogéno-alkyle en (C₁-C₆), alcoxy en (C₁-C₆) et halogéno-alcoxy en (C₁-C₆),
s signifie 0, 1, 2 ou 3,
n signifie 0, 1 ou 2,
D signifie 0, S ou NR¹¹,
à condition que V, X, Y et Z ne signifient pas simultanément hydrogène.

2. Amides d'acides N-(1,2,5-oxadiazol-3-yl)-, N-(1,3,4-oxadiazol-2-yl)-, N-(tétrazol-5-yl)- et N-(triazol-5-yl)-arylcarboxyliques acylés de la formule (I) selon la revendication 1, dans lesquels R signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₃-C₆), cycloalkyle en (C₃-C₆), cycloalkyle en (C₃-C₆)-alkyle en (C₁-C₆), halogénocycloalkyle en (C₃-C₆)-alkyle en (C₁-C₆), alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-COOR¹, alkyle en (C₁-C₆)-C(O)R¹, alkyle en (C₁-C₆)-CN, alkyle en (C₁-C₆)-SO₂OR¹, alkyle en (C₁-C₆)-CON(R¹)₂, alkyle en (C₁-C6)-SO₂N(R¹)₂, alkyle en (C₁-C₆)-NR¹COR¹, alkyle en (C₁-C₆)-NR¹SO₂R², COOR¹, CON(R¹)₂, ou phényle, hétéroaryle, hétérocyclyle ou benzyle, chacun substitués par s radicaux du groupe constitué par X, Y,
Z et V,
W signifie N ou CY,
X et Z signifient chacun indépendamment l'un de l'autre hydrogène, halogène, alkyle en (C₁-C₆), halogénoalkyle en (C₁-C₆), alcényle en (C₂-C₆), cycloalkyle en (C₃-C₆), halogénocycloalkyle en (C₃-C₆), OR¹, S(O)ₙR², SO₂N(R¹)₂, NR¹SO₂R², NR¹COR¹, alkyle en (C₁-C₆)-S(O)ₙR², alkyle en (C₁-C₆)-OR¹, ou
hétéroaryle, hétérocyclyle ou phényle, chacun substitués par s radicaux du groupe constitué par méthyle, éthyle, méthoxy, nitro, trifluorométhyle et halogène,
Y signifie hydrogène, alcényle en (C₂-C₆), COR¹, CO₂R¹, OCO₂R¹, NR¹CO₂R¹, C(O)N(R¹)₂, NR¹C(O)N(R¹)₂, OC(O)N(R¹)₂, C(O)N(R¹)OR¹, NR¹SO₂R², NR¹COR¹, OR¹, S(O)ₙR², SO₂N(R¹)₂, alkyle en (C₁-C₆)-S(O)ₙR², alkyle en (C₁-C₆)-OR¹, alkyle en (C₁-C₆)-OCOR¹, alkyle en (C₁-C₆)-CO₂R¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², N(R¹)₂, CH=NOR¹, alkyle en (C₁-C₆)-CH=NOR¹, alkyle en (C₁-C₆)-hétéroaryle, alkyle en (C₁-C₆)-hétérocyclyle, hétéroaryle ou hétérocyclyle, les 4 derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C6), alcoxy en (C₁-C₆)-alkyle en (C₁-C₄) et cyanométhyle, et l'hétérocyclyle portant n groupes oxo, V signifie hydrogène, Cl, OMe, méthyle ou éthyle,
R¹ signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆) -hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)ₙR⁴, N(R³)₂, NR³OR³, COR³, OCOR³, SCOR⁴, NR³COR³, NR³SO₂R4, CO₂R³, COSR⁴, CON(R³)₂ et alcoxy en (C₁-C₄)-alcoxycarbonyle en (C₂-C₆), et l'hétérocyclyle portant n groupes oxo,
R² signifie alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆) cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, les 21 derniers radicaux cités étant substitués par s radicaux du groupe constitué par cyano, halogène, nitro, rhodano, OR³, S(O)R⁴, N(R³)₂, NR³OR³, COR³, OCOR³, SCOR⁴, NR³COR³, NR³SO₂R⁴, CO₂R³, COSR⁴, CON(R³)₂ et alcoxy en (C₁-C₄)-alcoxycarbonyle en (C₂-C₆), et l'hétérocyclyle portant n groupes oxo,
R³ signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆),
R⁴ signifie alkyle en (C₁-C₆), alcényle en (C₂-C₆) ou alcynyle en (C₂-C₆), cycloalkyle en (C₃-C₆) ou cycloalkyle en (C₃-C₆)-alkyle en (C₁-C₆),
n signifie 0, 1 ou 2,
s signifie 0, 1, 2 ou 3,
Q signifie un radical Q1, Q2, Q3 ou Q4 : 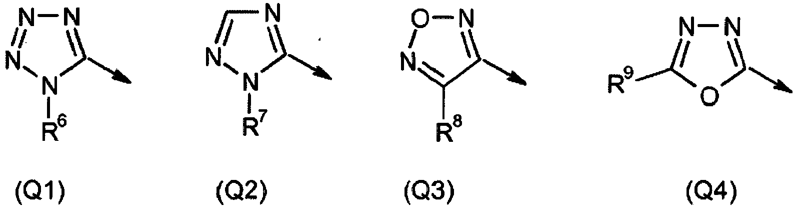
R⁶ et R⁷ signifient indépendamment l'un de l'autre alkyle en (C₁-C₆), halogéno-alkyle en (C₁-C₆), alcényle en (C₂-C₆), ces 3 radicaux mentionnés précédemment étant chacun substitués par s radicaux alcoxy en (C₁-C₆),
R⁸ signifie chlore, méthyle, méthoxyméthyle, amino ou acétylamino,
R⁹ signifie méthyle, éthyle, méthoxyméthyle ou méthoxyéthyle.

3. Amides d'acides N-(1,2,5-oxadiazol-3-yl)-, N-(1,3,4-oxadiazol-2-yl)-, N-(tétrazol-5-yl)- et N-(triazol-5-yl)-arylcarboxyliques acylés de la formule (I) selon la revendication 1 ou 2, dans lesquels
R signifie méthyle, ou phényle substitué respectivement par s radicaux du groupe constitué par X, Y et Z,
W signifie CY,
X signifie F, Cl, Br, méthyle, éthyle, cyclopropyle trifluorométhyle, méthoxy, méthoxyméthyle, méthoxyéthoxyméthyle, SMe ou SO₂Me,
Z signifie hydrogène, F, Cl, Br, I, méthyle, éthyle, trifluorométhyle, difluorométhyle, pentafluoroéthyle, méthylsulfonyle ou éthylsulfonyle,
Y signifie hydrogène, SMe, S(O)Me, SO₂Me, SEt, S(O)Et, SO₂Et, CH₂OMe, CH₂OEt, CH₂OCH₂CF₃, CH₂SMe, CH₂S(O)Me, CH₂SO₂Me, vinyle, C(O)Me, C(O)Et, C(O)cPr, CO₂Me, CHN=OMe, 4,5-dihydro-1,2-oxazol-3-yle, 5-méthyl-4,5-dihydro-1,2-oxazol-3-yle, 5-méthyl-4,5-dihydro-1,2-oxazol-3-yle, 5-cyanométhyl-4,5-dihydro-1,2-oxazol-3-yle, 4,5-dihydro-1,2-oxazol-5-yle, 3-méthyl-4,5-dihydro-1,2-oxazol-5-yle, 1H-pyrazol-1-yle, 1H-1,2,3-triazol-1-yle, 2H-1,2,3-triazol-2-yle, 1H-1,2,4-triazol-1-yle, pyrrolidin-2-on-1-yle, morpholin-3-on-4-yle, OMe, OEt, OnPr, OCH₂cPr, OCH₂CH₂F, OCH₂CH₂OMe ou OCH₂CH₂CH₂OMe,
V signifie hydrogène,
Q signifie un radical Q1, Q2, Q3 ou Q4 : 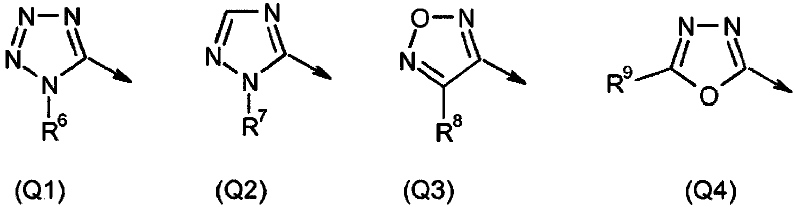
R⁶ signifie méthyle ou éthyle,
R⁷ signifie méthyle,
R⁸ signifie chlore ou méthyle,
R⁹ signifie méthyle,
s signifie 0, 1, 2 ou 3.

4. Agents herbicides, **caractérisés par** une teneur efficace du point de vue herbicide en au moins un amide d'acide N-(1,2,5-oxadiazol-3-yl)-, N-(1,3,4-oxadiazol-2-yl)-, N-(tétrazol-5-yl)- ou N-(triazol-5-yl)-arylcarboxylique acylé selon l'une quelconque des revendications 1 à 3.

5. Agents herbicides selon la revendication 4, en mélange avec des adjuvants de formulation.

6. Agents herbicides selon la revendication 5, contenant un herbicide supplémentaire.

7. Procédé de lutte contre des plantes indésirables, **caractérisé en ce qu'**une quantité efficace d'au moins un composé de la formule (I) selon l'une quelconque des revendications 1 à 3, ou d'un agent herbicide selon l'une quelconque des revendications 4 à 6 est appliquée sur les plantes ou à l'emplacement de la croissance végétale indésirable.

8. Utilisation de composés de la formule (I) selon l'une quelconque des revendications 1 à 3, ou d'agents herbicides selon l'une quelconque des revendications 4 à 6 pour lutter contre des plantes indésirables.

9. Utilisation selon la revendication 8, **caractérisée en ce que** les composés de la formule (I) sont utilisés pour lutter contre des plantes indésirables dans des cultures de plantes utiles.

10. Utilisation selon la revendication 9, **caractérisée en ce que** les plantes utiles sont des plantes utiles transgéniques.
